# EUROPEAN PATENT APPLICATION

(11) **EP 4 692 310 A1**
(43) Date of publication of application: **11.02.2026**
(21) Application number: 24781106.0
(22) Date of filing: 20.03.2024
(51) Int. Cl.: C12N 1/20, A61K 8/9789, A23L 33/105, A61K 36/23, A61P 17/00, A01G 7/06, C12R 1/11

(54) **NOVEL BACILLUS MEGATERIUM HYANGYAK-01 STRAIN AND USES THEREOF**

(30) Priority: 30.03.2023 KR 20230041710; 18.09.2023 KR 20230124308
(71) Applicant: Cosmax, Inc., Hwaseong-si, Gyeonggi-do 18622 (KR)
(72) Inventor: JO, Hyung Woo, Seongnam-si, Gyeonggi-do 13361 (KR); LEE, Dong Geol, Hwaseong-si, Gyeonggi-do 18496 (KR); KANG, Seung Hyun, Seoul 06285 (KR); LIM, Kyeong Mo, Daegu 41569 (KR); KIM, Min Chul, Daegu 41544 (KR); SHIN, Jae Ho, Daegu 41584 (KR)
(74) Representative: Lavoix
(86) International application number: PCT/KR2024/003498
(87) International publication number: WO 2024/205115

(57) **Abstract**

The present disclosure relates to a novel *Bacillus megaterium* HyangYak-01 strain and uses thereof.

The novel *Bacillus megaterium* HyangYak-01 strain according to one aspect exhibits effects of increasing diversity of microbiome species in soil from crop fields or improving physiochemical properties, promoting crop growth or biosynthesis of biologically active substances, and the like, and thus can be applied for various uses, such as improving soil condition, increasing crop cultivation efficiency, or enhancing crop efficacy.

## Description

### Technical Field

The present application is based on and claims priority to Korean Patent Application Nos. 10-2023-0041710, filed on March 30, 2023, and 10-2021-0046106, filed on April, 8, 2021, in the Korean Intellectual Property Office, the disclosures of which are incorporated by reference herein in their entirety.

Disclosed are a novel strain and uses thereof.

### Background Art

In recent years, overuse of organic and chemical fertilizers has caused many problems, such as soil contamination, failure in continuous cropping, imbalance of soil nutrients, and pest outbreaks, resulting in side effects including a decline in product quality and a decrease in farm incomes. Nitrogen (N), an important plant nutrient, has been supplied by the use of these chemical fertilizers, but the overuse of chemical fertilizers to maintain higher yields is problematic as it contradicts the principles of sustainable agriculture, which requires high productivity with low inputs, and also causes environmental issues.

To address these issues, biological methods are being actively studied as an alternative to chemical fertilizers to ensure safe food while minimizing the burden on the agricultural environment and ecosystems, and interest in and need for microbial fertilizers that can promote crop growth as an alternative are growing.

These microbial fertilizers have the advantage of not causing environmental issues of soil pollution such as salination, are excellent for maintaining ecosystem safety, and have the advantage of continuously improving crop productivity. With the increasing interest in eco-friendly and sustainable agriculture, research on agriculturally beneficial microorganisms has been steadily conducted.

Currently, various agriculturally useful microorganisms are commercially available, but there is little or no validation of their exact beneficial activity on crops. In addition, the number of chemical pesticides and fertilizers currently available is declining, and therefore there is a need to develop proven agriculturally useful microbial agents that can replace the chemical pesticides and fertilizers.

Accordingly, the inventors of the present disclosure have discovered a new strain of *Bacillus megaterium,* HyangYak-01, and confirmed that the strain has significantly excellent effects in increasing the diversity of microbiome species and improving physiochemical properties of soil from crop fields and promotes crop growth and biosynthesis of biologically active substances, and confirmed that *Centella asiatica* cultivated after treatment with the strain exhibits significantly excellent skin condition improvements and anti-inflammatory effects, thereby completing the present disclosure.

### Disclosure of Invention

### Technical Goal of the Invention

One aspect is to provide a novel strain of *Bacillus megaterium* HyangYak-01 (synonym: *Priestia megaterium* HyangYak-01).

Another aspect is to provide a microbial agent including the strain, a lysis solution or culture thereof, or an extract of the strain, the lysis solution, or the culture.

Another aspect is to provide a microbial fertilizer including the microbial agent.

Another aspect is to provide a method of cultivating a plant using the strain, the microbial agent, or the microbial fertilizer.

Another aspect is to provide a plant cultivated by the method.

Another aspect is to provide a cosmetic composition for improving a skin condition, an external skin application composition, or a food composition, each comprising *Centella asiatica* cultivated by the method or an extract thereof.

Another aspect is to provide a pharmaceutical composition for preventing or treating a skin inflammatory disease, the pharmaceutical composition including, as an active ingredient, *Centella asiatica* cultivated by the method or an extract thereof.

Another aspect is to provide a method of improving a skin condition of a subject or preventing, alleviating or treating a skin inflammatory disease of a subject, the method including administering an effective amount of the composition to a subject in need thereof.

Another aspect is to provide use for preparation of the strain, a lysis solution or culture thereof, or an extract of the strain, the lysis solution, or the culture.

### Solution to Problem

One aspect provides a *Bacillus megaterium* strain.

The scientific name *Priestia megaterium* may be used as a synonym for *Bacillus megaterium.* In this regard, the strain may be also referred to as a *Priestia megaterium* strain.

The strain may be isolated. In the present specification, the term "isolated" refers to something that is not in its natural state but has been artificially isolated from nature or other sources and made available for use. The strain may be isolated from soil. Specifically, the strain may be isolated by methods of performing serial dilution on the rhizosphere soil of a crop cultivation site, inoculating diluted soil onto tryptic soy broth (TSB) agar medium or Reasoner's 2A (R2A) agar medium, and then pure culturing cultured colonies.

The strain may belong to the Bacillus species (*Bacillus sp*.)*.*

The strain may include 16S rRNA comprising a base sequence of SEQ ID NO: 1. Specifically, the strain may include 16S rRNA comprising a base sequence having at least about 95 %, at least about 96 %, at least about 97 %, at least about 98 %, at least about 99 %, at least about 99.5 %, or at least about 99.9 % sequence identity to SEQ ID NO: 1. The strain may include 16S rRNA having the base sequence of SEQ ID NO: 1.

In the present specification, the term "sequence identity" refers to a degree of identity between amino acid residues or bases in two sequences in a particular comparison region after aligning the sequences as closely as possible. The sequence identity may be confirmed by methods known in the art. The percentage of sequence identity may be determined by sequence comparison programs known in the art, and examples of such programs are BLASTN (NCBI), CLC Main Workbench (CLC bio), MegAlignTM (DNASTAR Inc), etc.

The *Bacillus megaterium* strain may be a strain deposited under Accession No. KCCM13074P. The strain deposited under the Accession No. above may be a *Bacillus megaterium* HyangYak-01 (synonym: *Priestia megaterium* HyangYak-01) strain.

The strain may have effects such as improving soil conditions, promoting plant growth, or promoting biosynthesis of biologically active substances in plants. Specifically, the plants may be crops, i.e., plants cultivated for human use, but are not limited thereto. More specifically, the plants may include at least one selected from the group consisting of all monocotyledonous plants and dicotyledonous plants, or all herbaceous plants and woody plants, including plants (e.g., ornamental plants, field plants, mountain plants, etc.) grown in dry environment (e.g., fields, gardens, pots, mountains, etc.), waterlogged or water-grown crops (e.g., paddy crops including rice), hydroponic crops, and aquatic plants. For example, the plants may include at least one selected from the group consisting of *Centella asiatica, Angelica gigas, Platycodon grandiflorum, Codonopsis lanceolata, Potentilla chinensis, Isodon japonicus,* and *Glycyrrhiza uralensis,* but are not limited thereto.

In the present specification, the term *"Centella asiatica* (scientific name)" is a perennial creeping medicinal plant that mainly grows in South Asia, and grows in clusters in lowland wetlands in Jeju Island and the south-central region of South Korea. The main morphological characteristics of the *Centella asiatica* are as follows: the main stem grows horizontally, with about two degenerated scale-like leaves near the nodes where the roots emerge; the leaves are densely arranged at the nodes and kidney-shaped, with a diameter of about 2 to 5 centimeters (cm); the surface is glossy, with blunt teeth along the margins; and the petiole is about 4 to 20 cm long. However, the characteristics are not limited thereto.

In the present specification, the term *"Angelica gigas"* refers to a perennial herb belonging to the family *Apiaceae,* distributed in Korea, China, and Japan. For reference, it is also called 'cham-dang-gwi' and 'sin-gam-chae'. The main morphological characteristics of the *Angelica gigas* are as follows: the height is about 1 to 2 meters (m); the whole stem is purple; and the flower is purple, but the characteristics are not limited thereto.

In the present specification, the term *"Platycodon grandiflorum* (scientific name)" refers to a perennial plant of a species *Platycodon grandiflorus* of the family Campanulaceae. It is also known as balloon flower, and its other Chinese names are pronounced 'hwa-sang-mo,' 'myeong-yeob-chae,' and 'do-lab-gi'. The main morphological characteristics of the *Platycodon grandiflorum* are as follows: the height is about 40 to 100 cm; the roots are thick; the stem grows as one or in several branches; a white sap comes out when the main stem is cut; and the leaves are alternate or opposite, about 5 to 12 cm long, ovate-shaped, narrow at both ends, and serrated edges. However, the characteristics are not limited thereto.

In the present specification, the term *"Codonopsis lanceolata* (scientific name)" refers to a perennial climbing plant that belongs to the family Campanulaceae and is native to Korea and distributed in Japan, China, Manchuria, and Ussuri. Its other Chinese names are pronounced 'sa-sam ' and 'san-hae-ra'. The main morphological characteristics of the *Codonopsis lanceolata* are as follows: the length is about 2 to 5 m; the leaves appear clustered together at the tips of short branches, with about 4 leaves, and have a long oval shape measuring about 3 to 10 cm in length and 1.5 to 4 cm in width; the leaf margins are smooth, with a green surface and a powdery white underside; the flowers have a light green outer surface with brownish spots on the inner side; the roots of the *Codonopsis lanceolata* are thick like those of the *Platycodon grandiflorum*; and a white sap emerges when the vine is cut. However, the characteristics are not limited thereto.

In the present specification, the term *"Potentilla chinensis* (scientific name)" refers to a perennial herbaceous plant belonging to the family Rosacea, and is also known as 'wei-ling-chae' and 'dong-nok-pul'. The main morphological characteristics of the *Potentilla chinensis* are as follows: it flourish to a height of about 30 to 60 cm; the roots are thick; the leaves are pinnately compound leaves with a dark green upper surface and a white underside covered with dense hairs; and the flowers are yellow. However, the characteristics are not limited thereto.

In the present specification, the term *"Isodon japonicus* (scientific name)" refers to a perennial plant belonging to the family *Lamiaceae* of dicotyledonous lamiales. The main morphological characteristics of the *Isodon japonicus* are as follows: it grows to a height of about 50 to 100 cm and has underground stems; the stems are erect with square ridges and downward-pointing hairs and branch profusely; the leaves are opposite, broad ovate in shape, about 6 to 15 cm in long and about 3.5 to 8 cm in width, and have serrated edges; the base suddenly narrows to form wing=like structures on the leaf stalk; and the flowers are pale purple and arranged in a raceme. However, the characteristics are not limited thereto.

In the present specification, the term *"Glycyrrhiza uralensis* (scientific name)" refers to a perennial herbaceous plant belonging to the Fabaceae family of the Rosales order of dicotyledonous plants. The main morphological characteristics of the *Glycyrrhiza uralensis* are as follows: the roots are reddish-brown and grow deep into the ground; the stems are branched and grow straight to about 1 m and covered with dense white hairs showing a greyish-white appearance; granular dots are scattered along the stems; the leaves are alternate and odd-pinnate compound leaves, with small leaflets counting about 7 to 17 leaves, egg-shaped with pointed tips, and the length of the small leaflets is about 2 to 5 cm, with a width of about 1 to 3 cm and both surfaces covered with white hairs and glandular dots without teeth; and the flowers are about 1.4 to 2.5 cm long, purple, and arranged in racemes in the leaf axils. However, the characteristics are not limited thereto.

The improving of soil conditions may include increasing diversity of microbial species in the soil microbiome or improving the physiochemical properties of soil.

The physiologically active substance of the plant may include any one or more selected from the group consisting of chlorophyll, madecassic acid, and asiatic acid.

In the present specification, the term "microbiome" may refer to an ecological environment consisting of microorganisms.

In the present specification, the term "physiochemical properties" may refer to physical or chemical properties representing properties of soil (e.g., pH, amount of organic matters, amount of effective phosphorus, amount of exchangeable cations, electrical conductivity, etc.). In the present specification, the term "improving physiochemical properties of soil" may refer to improving physical or chemical properties of soil to a state more suitable for cultivation or growth of plants. Specifically, the improving of physiochemical properties of soil may include any one or more selected from the group consisting of: maintaining soil pH at slightly acidic (pH 6.0 to 6.5) or mildly acidic (pH 6.5 to 7.0) levels; promoting decomposition of organic matters in soil; inducing an increase in the amount of exchangeable cations in soil; maintaining electrical conductivity of soil at normal levels (about 2 ds m⁻¹ or less or about 1 to 2 ds m⁻¹); and maintaining the amount of effective phosphorus in soil at normal levels (about 400 to 500 mg kg⁻¹).

In the present specification, the term "growth" may refer to an increase in the size or weight of an organism due to an increase in the number of cells constituting the organism.

In the present specification, the term "biologically active substance" may refer to an organic substance or inorganic ion that affects a biological life phenomena even in very small amounts.

In the present specification, the term "biosynthesis" may refer to a metabolism of an organic substance synthesized by cellular processes within an organism.

According to an embodiment, it was confirmed that the diversity of microbiome species significantly increased in soil from crop fields treated with the *Bacillus megaterium* HyangYak-01 strain, as compared to untreated soil. Therefore, when the strain is applied to soil, the soil will have increased species diversity of microbiome such that it becomes healthier for crop cultivation, and as a result, the growth of crops cultivated in the soil may be further promoted.

According to an embodiment, in soil from crop fields treated with the *Bacillus megaterium* HyangYak-01 strain, as compared to untreated soil, the physiochemical properties of the soil may be further improved (in terms of: maintaining soil pH at slightly acidic or mildly acidic; promoting decomposition of organic matters in soil; increasing the amount of exchangeable cations in soil; and maintaining electrical conductivity of soil and the amount of effective phosphorus in soil, at normal levels) such that the soil becomes healthier for crop cultivation, and as a result, the growth of crops cultivated in the soil may be further promoted.

According to an embodiment, in plants cultivated using the *Bacillus megaterium* HyangYak-01 strain, the root length, root weight (fresh weight and dry weight), leaf length, leaf weight (fresh weight and dry weight), leaf width, number of leaves, and chlorophyll amount were all significantly increased compared to an untreated control group. Therefore, the strain may exhibit an effect of significantly promoting or enhancing plant growth (e.g., any one or more selected from root length, root weight, leaf length, leaf weight, number of leaves, and chlorophyll amount).

According to an embodiment, in *Centella asiatica* cultivated using the *Bacillus megaterium* HyangYak-01 strain, it was confirmed that the amount of any one or more selected from the group consisting of chlorophyll, madecassic acid, and asiatic acid in the *Centella asiatica* was significantly increased compared to the untreated control group. Therefore, the strain may exhibit an effect of enhancing biologically active substances (e.g., any one or more selected from the group consisting of chlorophyll, madecassic acid, and asiatic acid) in plants, i.e., an effect of promoting biosynthesis of biologically active substances in plants.

The strain may exhibit any one or more of the following activities: production activity of a root growth-promoting hormone (indole-3-acetic acid: IAA); nitrogen fixation activity; phosphate solubilization activity; urea hydrolysis enzyme (urease) activity; and denitrification activity.

In the present specification, the term "nitrogen fixation" may refer to the action of converting nitrogen gas molecules (N) in the air into nitrogen compounds, such as ammonia (NH), which can be utilized by plant cells.

In the present specification, the term "phosphate solubilization" may refer to the action of converting insoluble phosphate into ion forms, such as H₂PO₄⁻ or HPO₄²⁻, which can be easily utilized by plants or microorganisms.

In the present specification, the term "urea hydrolysis enzyme (urease) activity" may refer to activity that exhibits the same effect as urase, i.e., activity of hydrolyzing urea, and more specifically, activity of hydrolyzing urase exhibited by a strain producing urase. Therefore, the term "urase activity" may be used interchangeably with terms such as "urea-hydrolyzing activity," "urase-producing activity," and the like.

In the present specification, the term "denitrification" may refer to the action of reducing nitrate nitrogen into nitrogen gas (N₂).

The strain may include any one or more genes selected from the group consisting of a gene related to production of root growth-promoting hormone as a gene involved in the crop growth promotion activity, a gene related to the nitrogen fixation, a gene related to the phosphate solubilization, a gene related to the urase, and a gene related to the denitrification. According to an embodiment, the strain may include any one or more genes selected from the group consisting of *gatA, puuPER, aldH, ytnP, phoP, phoR, phoH, nifS, narT, nirCQ, norRMG, ureGFECBDARI,* and *ureDGFECBA.*

Another aspect provides a lysis solution or culture of the *Bacillus megaterium* strain, or an extract of the strain, the lysis solution, or the culture.

Specific details of the strain are as described above.

In the present specification, the term "lysis solution" may be used interchangeably with "lysate", and may refer to a product obtained by disrupting the cell wall of the strain by chemical or physical force. The lysis solution may contain the lysis solution itself or a concentrate or lyophilized product thereof.

In the present specification, the term "culture" may be used interchangeably with the terms "culture solution," culture supernatant," "conditioned culture solution," or "conditioned medium," and may refer to the entire medium containing the *Bacillus megaterium* strain or metabolites thereof, extra nutrients, etc., obtained by culturing the strain for a certain period of time in a medium capable of providing nutrients to enable the strain to grow and survive in vitro. In addition, the culture may refer to a culture solution from which bacterial cells have been removed from a culture solution of bacterial cells obtained by culturing the strain. Meanwhile, the liquid from which the bacterial cells have been removed from the culture solution is also called "supernatant", which may be obtained by allowing the culture solution to stand still for a certain period of time and collecting only the liquid in the upper layer, excluding the portion that has settled in the lower layer, by removing the bacterial cells through filtration, or by centrifuging the culture solution to remove the precipitates at the bottom and collecting only the liquid at the top. The term "bacterial cell" refers to the strain of the present disclosure itself, and may include a strain itself isolated and selected from a sample or the like, or a strain isolated from a culture solution by culturing the strain. The bacterial cell may be obtained by centrifuging the culture solution and collecting the portion that has precipitated to the lower layer, or the bacterial cell precipitates to the lower layer of the culture solution by gravity and can therefore be obtained by leaving the culture solution still for a certain period of time and removing the liquid from the top.

The culture may include a culture solution itself obtained by culturing a strain, a concentrate or freeze-dried product thereof, or a culture supernatant obtained by removing a strain from a culture solution, or a concentrate or freeze-dried product thereof.

A culture medium and culture conditions for culturing the *Bacillus megaterium* strain may be appropriately selected or modified by those skilled in the art. For example, the culture solution was obtained by culturing in a medium (e.g., R2A medium) at a temperature of about 20 to 40 °C (e.g., about 30 °C) for a certain period of time, such as about 30 to 80, 40 to 80, or 48 to 72 hours.

In an embodiment, the culture supernatant of the strain may be obtained by the removing of bacterial cells through centrifugation or filtration of the culture solution of the strain.

In another embodiment, the concentrate may be obtained by the concentrating of the culture solution of the strain itself, or the supernatant obtained by centrifugation or filtration of the culture solution.

In the present specification, the term "an extract of the strain, the lysis solution, or the culture" refers to the extract from the strain, lysis solution, or culture, or concentrate of the foregoing, and may include an extract solution, a diluted solution or concentrated solution of the extract solution, a dried product obtained by drying the extract solution, or a crude product or purified product of the foregoing, as well as a fraction obtained by fractionation of the foregoing.

The strain, the lysis solution or culture thereof, or the extract of the strain, the lysis solution, or the culture may be biologically pure. The strain may be obtained by pure culture.

The terms or elements mentioned in the 'lysis solution, culture, or extract' that are the same as those mentioned in the description of the 'strain' are understood to be the same as those mentioned in the description of the 'strain'.

Another aspect provides uses of: the *Bacillus megaterium* strain; a lysis solution or culture thereof; or an extract of the strain, the lysis solution, or the culture.

Another aspect provides a microbial agent and use thereof, the microbial agent including, as an active ingredient, the *Bacillus megaterium* strain, a lysis solution or culture thereof, or an extract of the strain, the lysis solution, or the culture.

Another aspect provides a microbial fertilizer and use thereof, the microbial fertilizer including the microbial agent as an active ingredient.

Regarding the uses or the microbial agent or microbial fertilizer, the *Bacillus megaterium* strain may be the *Bacillus megaterium* strain according to one aspect. That is, the strain may be the *Bacillus megaterium* HyangYak-01 strain.

The uses of: the strain; the lysis solution or culture thereof; or the extract of the strain, the lysis solution, or the culture; or the microbial agent or microbial fertilizer may include improving soil conditions, promoting plant growth, or promoting biosynthesis of biologically active substances. In addition, the strain; the lysis solution or culture thereof; or the extract of the strain, the lysis solution, or the culture; or the microbial agent or microbial fertilizer may be used for cultivating plants including crops (e.g., *Centella asiatica*). Specific details of the plant are as described above.

The form of the microbial agent is not significantly limited, but may be provided in the form of a microbial pesticide or microbial fertilizer, and more preferably in the form of a seed-coating agent, a soil conditioner, a compost accelerator, a foliar spray, or a drip irrigation spray. Specifically, the microbial agent may be formulated by conventional methods for the aforementioned uses, and may be prepared in the form of a dry powder or a liquid fertilizer. Specifically, the microbial agent may be prepared in a liquid fertilizer form, and an extender may be added thereto to be prepared in a powder form, or the powder form may be formulated to be granulated. However, the formulation is not specifically limited. Preferably, the microbial agent may be formulated as a biological fertilizer to replace chemical fertilizers, and thus may be formulated as a biological fertilizer to overcome the limitations of chemical fertilizer supply in eco-friendly organic farming.

The microbial agent may be prepared by adding additives, such as an additive, an extender, a nutrients, and the like, to the strain, the lysis solution or culture thereof, or the extract of the strain, the lysis solution, or the culture. Here, the additive may include at least one selected from the group consisting of polycarboxylate, sodium lignosulfonate, calcium lignosulfonate, sodium dialkyl sulfosuccinate, sodium alkyl aryl sulfonate, polyoxyethylene alkylphenyl ether, sodium polyphosphate, polyoxyethylene alkylaryl phosphoric ester, polyoxyethylene alkylaryl ether, polyoxyethylene alkylaryl polymer, polyoxyalkylene alkylphenyl ether, polyoxyethylene nonylphenyl ether, sodium sulfonate naphthalene formaldehyde, Triton 100, and Tween 80, the extender and nutrient may include one or at least two selected from the group consisting of skim milk (medium), soybean powder, rice, wheat, yellow clay, diatomaceous earth, bentonite, dextrin, glucose, and starch, and a disintegrant may include at least one selected from the group consisting of bentonite, talc, dialite, kaolin, and calcium carbonate.

In the present specification, the term "microbial fertilizer" refers to a product that has the same effect as a fertilizer by: pure culturing microorganisms that have a beneficial effect on the growth of plants such as crops, mixing the microorganisms with seeds, and then sowing the mixture; or by inoculating the microorganisms directly into the soil.

The microbial fertilizer may have a formulation selected from the group consisting of a seed-coating agent, a seed-soaking agent, a soil conditioner, a compost accelerator, a foliar spray agent, and a drip irrigation agent, but is not limited thereto, and may be prepared or formulated by conventional methods known in the art for the aforementioned uses.

The microbial fertilizer may include a carrier or excipient used in conventional plant pesticides, and as the carrier or excipient, bentonite, surfactants, talc, zeolite, etc. may be used.

The terms or elements mentioned in the 'use, microbial agent, or microbial fertilizer' that are the same as those mentioned in the description of the 'strain' or 'lysis solution, culture, or extract' are understood to be the same as those mentioned in the description of the 'strain' or 'lysis solution, culture, or extract'.

Another aspect provides a method of cultivating plants, the method including treating any one or more of soil, plants, and plant seeds, with the *Bacillus megaterium* strain, microbial agent, or microbial fertilizer.

The method may be for improving soil conditions, promoting plant growth, or promoting biosynthesis of biologically active substances in plants.

Accordingly, another aspect provides a method of improving soil conditions, promoting plant growth, or promoting biosynthesis of biologically active substances in plants, the method including treating any one or more of soil, plants, and plant seeds with the *Bacillus megaterium* strain, microbial agent, or microbial fertilizer.

The plant may be at least one selected from the group consisting of *Centella asiatica, Angelica giga, Platycodon grandiflorum, Codonopsis lanceolata, Potentilla chinensis, Isodon japonicus,* and *Glycyrrhiza uralensis,* but is not limited thereto, and a detailed description of the plant is as described above.

Treatments used in the treating may generally include commonly used methods such as scattering (e.g., spraying, misting, atomizing, powder scattering, granule scattering, water surface application, continuous application, etc.), soil application (e.g., mixing, irrigation, sowing, etc.), surface application (e.g., coating, smearing, covering, etc.), immersion, poisoning, smoking, etc., but are not limited to these methods. In an embodiment, regarding the method, the treating of the plant seeds with the strain, microbial agent, or microbial fertilizer may be performed by mixing the strain, microbial agent, or microbial fertilizer with the plant seeds and then sowing the mixture.

The amount of the *Bacillus megaterium* strain, microbial agent, or microbial fertilizer may be appropriately determined based on factors such as formulation, purpose, application method, application site, application area, crop yield, soil condition, plant characteristics or condition, etc. For example, the effective amount of microorganisms treated by the method may be included in the number of microorganisms of about 1 to 1×10¹⁰⁰ microorganisms per cultivated land area (m²) (or colony forming unit (CFU)), but is not limited thereto.

Another aspect provides a plant cultivated by the method.

The plant may be at least one selected from the group consisting of *Centella asiatica, Angelica giga, Platycodon grandiflorum, Codonopsis lanceolata, Potentilla chinensis, Isodon japonicus,* and *Glycyrrhiza uralensis,* but is not limited thereto, and a detailed description of the plant is as described above.

According to an embodiment, the plant cultivated by the method may exhibit enhanced growth compared to plants not cultivated by the above method (e.g., plants cultivated without treatment with the *Bacillus megaterium* strain, microbial agent, or microbial fertilizer), resulting in further promoted growth leading to further increased growth levels (e.g., increased level in any one or more selected from root length, root weight, leaf length, leaf weight, leaf width, number of leaves, and chlorophyll amount), or resulting in further promoted biosynthesis of biologically active substances leading to further increased amount of biologically active substances (e.g., at least one selected from chlorophyll, madecassic acid, and asiatic acid).

According to an embodiment, in the *Centella asiatica* cultivated by the method, the amount of chlorophyll may the increased by about 5 to 70, 5 to 35, 10 to 60, 10 to 30, 20 to 60, 20 to 30, or 30 to 60 %, the amount of madecassic acid may be increased by about 1 to 50, 5 to 50, 10 to 50, 30 to 50, 1 to 40, 5 to 40, 10 to 40, 30 to 40, 1 to 30, 5 to 30, or 10 to 30 %, and the amount of asiatic acid may be increased by about 1 to 60, 5 to 60, 10 to 60, 30 to 60, 1 to 50, 5 to 50, 10 to 50, 30 to 50, 1 to 40, 5 to 40, 10 to 40, 30 to 40, 1 to 30, 5 to 30, or 10 to 30 %, compared to the plant cultivated without treatment with the *Bacillus megaterium* strain, microbial agent, or microbial fertilizer.

The terms or elements mentioned in the 'method or plant' that are the same as those mentioned in the 'strain', 'lysis solution, culture, or extract', or 'use, microbial agent, or microbial fertilizer' are understood to be the same as those mentioned in the previous description of the 'strain', 'lysis solution, culture, or extract', or 'use, microbial agent, or microbial fertilizer'.

Another aspect provides use of *Centella asiatica* cultivated by the method or an extract thereof.

Another aspect provide a cosmetic composition, an external skin application composition, or a food composition, each of which includes, as an active ingredient, the *Centella asiatica* cultivated by the method or an extract thereof, and also provides uses of the compositions.

The uses may include improving a skin condition. The improving of a skin condition may include skin barrier strengthening or anti-inflammation (or skin anti-inflammation or suppression (alleviation) of skin inflammation).

The extract of *Centella asiatica* (*Centella asiatica* extract) may include the extract itself and all types of extract formulations that can be formed using the extract, such as an extract obtained by extracting the *Centella asiatica,* a diluted solution or concentrated solution of the extract, a dried product obtained by drying the extract, a crude product or purified product of the extract, or a mixture of the foregoing.

The extract of *Centella asiatica* (*Centella asiatica* extract) may be extracted from natural, hybrid, or mutant plants of the *Centella asiatica,* and can also be extracted from plant tissue cultures.

Extraction methods for *Centella asiatica* to prepare the extract of the *Centella asiatica* (*Centella asiatica* extract) are not particularly limited, but may include those commonly used in the art. Non-limiting examples of the extraction methods include hot water extraction, ultrasonic extraction, filtration, reflux extraction, etc., which may be performed alone or in combination with two or more methods. Types of an extraction solvent used for the extraction are not particularly limited, and may include any solvent known in the art. Non-limiting examples of the extraction solvent include, but are not limited to, at least one selected from the group consisting of water, anhydrous or low-grade alcohols of C1 to C4, a mixed solvent of the aforementioned water and low-grade alcohols, acetone, 1,3-butylene glycol, ethyl acetate, chloroform, ethanol, and the like, which may be used alone or in a mixture of two or more. In the present disclosure, ethanol (e.g., about 60 to 100, 70 to 100, 60 to 80, or 70 % ethanol) may be used as the extraction solvent. The solvent may be used to extract the *Centella asiatica* once or more to prepare a solvent extract, and the solvent extract may be distilled under reduced pressure and then freeze-dried or spray-dried to prepare a dried extract. In addition, extracts obtained by heat extraction or cold extraction may be filtered to remove suspended solid particles, such as by filtering particles out using nylon or filtering by freeze filtration, and then used as it is or dried by freeze-drying, hot-air drying, spray drying, etc.

According to an embodiment, the *Centella asiatica* cultivated by the method or an extract thereof, or the compositions may exhibit any one or more of the following characteristics: inducing an increase in the expression of skin barrier strengthening factors, such as filaggrin (FLG), claudin-1 (CLD1), or a combination thereof; and inducing inhibition of the expression of pro-inflammatory cytokines.

In the present specification, the term "pro-inflammatory cytokine" may refer to a cytokine that causes or promotes inflammation or exacerbates disease accordingly.

The pro-inflammatory cytokine may include interleukin-1 alpha (IL-1α), interleukin-1 beta (IL-1β), or a combination thereof, but is not limited thereto.

Therefore, the *Centella asiatica* or the extract thereof, or the compositions may exhibit excellent effects of improving a skin condition (e.g., skin barrier strengthening effects) or anti-inflammatory effects (or skin anti-inflammation or inhibition (or alleviation) of skin inflammation) by significantly increasing the expression of skin barrier strengthening factors or significantly inhibiting the expression of pro-inflammatory cytokines that induce inflammation (or skin inflammation).

In the present specification, the term "expression" may refer to gene expression, or may refer to all or part of a process by which various proteins that constitute living organisms are formed from genetic information, i.e., genes, that constitute DNA. For example, the gene expression may include expression of DNA, RNA (e.g., mRNA), protein, or a combination thereof, and more specifically, may include transcription of DNA into RNA, translation of RNA into protein, or a combination thereof. For example, the term "increased expression" as used in the present specification may refer to an increase in the expression of a protein or DNA or RNA (e.g., mRNA) encoding the same.

The "skin barrier strengthening" may refer to any action that enhances the function of the skin barrier located on the outermost layer of the skin, which prevents moisture and nutrient loss, or any action that inhibits or restores damage to the skin barrier function.

The impaired skin barrier function may refer to any change in the skin that occurs as a result of a decreased or impaired function of the skin barrier. For example, the any change may include increased skin wrinkles, dryness, dermatitis, atopic dermatitis, allergic dermatitis, acne, etc.

The "anti-inflammation" may refer to any action that inhibits or interferes with inflammatory responses.

Another aspect provides a pharmaceutical composition for preventing, ameliorating, or treating a skin inflammatory disease, the pharmaceutical composition including, as an active ingredient, the *Centella asiatica* cultivated by the method or an extract thereof.

The skin inflammatory disease may be any one selected from the group consisting of dermatitis, atopic dermatitis, pruritus, eczematous skin disease, dry eczema, erythema, urticaria, psoriasis, drug rash, and acne.

The *Centella asiatica* cultivated by the method or an extract thereof, or the pharmaceutical composition may prevent, ameliorate, or treat the skin inflammatory disease by significantly inhibiting the expression of pro-inflammatory cytokines that cause inflammation (or skin inflammation).

In the present specification, the term "prevention" may include inhibiting the occurrence of a disease. In the present specification, the term "treatment" may include inhibition, reduction, or elimination of the development of a disease. In the present specification, the term "alleviation" may refer to parameters associated with relief or treatment of a condition, and for example, may refer to any action that at least reduces severity of a symptom.

The composition may include, based on the total weight of the composition, the *Centella asiatica* cultivated by the method of an extract thereof in an amount in a range of about 0.001 wt% to 80 wt%, for example, about 0.01 wt% to 60 wt%, about 0.01 wt% to 40 wt%, about 0.01 wt% to 30 wt%, about 0.01 wt% to 20 wt%, about 0.01 wt% to 10 wt%, about 0.01 wt% to 5 wt%, about 0.05 wt% to 60 wt%, about 0.05 wt% to 40 wt%, about 0.05 wt% to 30 wt%, about 0.05 wt% to 20 wt%, about 0.05 wt% to 10 wt%, about 0.05 wt% to 5 wt%, about 0.1 wt% to 60 wt%, about 0.1 wt% to 40 wt%, about 0.1 wt% to 30 wt%, about 0.1 wt% to 20 wt%, about 0.1 wt% to 10 wt%, or about 0.1 wt% to 5 wt%. Here, when the amount of the *Centella asiatica* cultivated by the method or an extract thereof is beyond the ranges above, the effects of improving a skin condition, such as skin barrier strengthening effect or the anti-inflammatory effect, may be reduced or may not be sufficiently exhibited.

In the present specification, the term "including as an active ingredient" refers to adding of the *Centella asiatica* cultivated by the method or an extract thereof in an effective amount that is sufficient to exhibit the aforementioned effects, and refers that various ingredients are added as secondary ingredients and then formulated in various types for drug delivery and stabilization.

The composition may be in a liquid state or a dry state. In an embodiment, the composition may be in the form of dry powder.

Drying methods for preparation of the composition in a dry state may include methods generally used in the art, and are not particularly limited. Non-limiting examples of the drying methods include an air-drying method, a natural drying method, a spray drying method, a freeze drying method, and the like. These methods may be used alone or in combination of at least two methods.

The composition may include an effective amount of an additive sufficient to reduce deterioration of the *Centella asiatica* cultivated by the method or an extract thereof. The additive may be, for example, a binding agent, but is not limited thereto.

The composition may further include a carrier that is cosmetically acceptable, pharmaceutically acceptable, or food acceptable. The composition may be formulated with a carrier and provided as a cosmetic, a drug, a food additive, and the like.

The cosmetic composition may additionally include ingredients and functional additives commonly used in cosmetic compositions, in addition to the effective ingredients described herein, and may include, for example, conventional auxiliary agents, such as antioxidants, stabilizers, solubilizers, surfactants, dispersants, emulsifiers, preservatives, vitamins, pigments, and fragrances, and carriers.

The cosmetic composition is not particularly limited to a specific formulation, and the formulation thereof may be appropriately selected depending on the intended purpose. The cosmetic composition may have, for example, a solubilized formulation, an emulsified formulation, or a dispersed formulation. The cosmetic composition may be, for example, formulated into a softening toner, a nourishing toner, a massage cream, a nourishing cream, an essence, a pack, a gel, an ampoule, or an adhesive type cosmetics.

The cosmetic composition may be a cosmetic raw material composition used in the preparation of cosmetics. In addition, the cosmetic composition may be a cosmetic composition having a final cosmetic formulation.

The external skin application may be formulated into a cream, a gel, an ointment, a skin emulsifier, a skin suspension, a transdermal delivery patch, a drug-containing bandage, a lotion, or a combination thereof. For the external skin application, ingredients used in ordinary cosmetics or external skin applications, such as water-based ingredients, oil-based ingredients, oil-based ingredients, powdered ingredients, alcohols, moisturizers, thickeners, ultraviolet ray absorbents, whitening agents, preservatives, antioxidants, surfactants, flavoring agents, colorants, various skin nutrients, or a combination thereof, may be suitably blended as needed. For the external skin application, a sequestrant, such as disodium edetate, trisodium edetate, trisodium citrate, sodium polyphosphate, sodium metaphosphate, gluconic acid, or the like, caffeine, tannin, verapamil, a licorice extract, glabridin, a hot water extract of caline fruit, various herbal medicines, tocopherol acetate, glycyrrhizic acid, tranexamic acid, a derivative thereof, or a medicine of a salt or the like thereof, vitamin C, magnesium ascorbyl phosphate, ascorbyl glucoside, albutin, kojic acid, or sugar, such as glucose, fructose, trehalose, or the like, may be suitably blended.

The food composition may include a health functional food composition. In this case, the food composition may be formulated into a typical health functional food formulation known in the art.

The health functional food composition may use the *Centella asiatica* cultivated by the method or an extract thereof, or may be used in combination with other foods or food ingredients, and may be used appropriately by conventional methods. A mixing amount of the active ingredient may be appropriately determined depending on the purpose of use (prevention, health or therapeutic treatment). Types of the health functional food are not particularly limited. Among the types of the health functional food, a beverage composition may include, as additive ingredients, various flavoring agents, sweetening agents, or natural carbohydrates as in general beverages. The natural carbohydrates may include monosaccharides, such as glucose and fructose, disaccharides, such as maltose and sucrose, polysaccharides, such as dextrin and cyclodextrin, and sugar alcohols, such as xylitol, sorbitol, and erythritol. The sweetening agents may use natural sweetening agents, such as thaumatin and a stevia extract, synthetic sweetening agents, such as saccharin and aspartame, and the like. The food composition may also contain a nutrient, a vitamin, an electrolyte, a flavoring agent, a colorant, pectic acid and a salt thereof, alginic acid and a salt thereof, organic acid, a protective colloidal thickener, a pH adjuster, a stabilizer, a preservative, glycerin, alcohol, and a carbonizing agent used in carbonated beverages, or a combination thereof. The food composition may also include natural fruit juice and fruit flesh for preparing fruit juice beverage and vegetable beverage, or a combination thereof.

The pharmaceutical composition may further include a diluent or a carrier. The diluent may be lactose, corn starch, soybean oil, microcrystalline cellulose, mannitol, or a combination thereof. The carrier may include an excipient, a disintegrant, a binding agent, a glidant, or a combination thereof. The excipient may include microcrystalline cellulose, lactose, low-substituted hydroxy cellulose, or a combination thereof. The disintegrant may include carboxymethyl cellulose calcium, sodium starch glycolate, anhydrous calcium hydrogen phosphate, or a combination thereof. The binding agent may include polyvinyl pyrrolidone, low-substituted hydroxypropyl cellulose, hydroxypropyl cellulose, or a combination thereof. The glidant may include magnesium stearate, silicon dioxide, talc, or a combination thereof.

The pharmaceutical composition may be formulated for oral or parenteral administration. The formulation for oral administration may include granules, powders, liquids, tablets, capsules, dry syrups, and the like. The formulation for parenteral administration may include injections, ointments, and the like.

The terms or elements mentioned in the 'use or composition' that are the same as those mentioned in the 'strain', 'lysis solution, culture, or extract', 'use, microbial agent, or microbial fertilizer', or 'method or plant' are understood to be the same as those mentioned in the previous description of the 'strain', 'lysis solution, culture, or extract', 'use, microbial agent, or microbial fertilizer', or 'method or plant'.

Another aspect provides a method of preventing, ameliorating, or treating a condition of a subject, the method including administering an effective amount of the composition to a subject in need thereof.

The condition of a subject may be a skin-related condition such as skin barrier dysfunction or skin inflammation.

In the present specification, the terms "administering", "introducing", and "grafting" may be used interchangeably, and may be construed as arranging a composition according to an embodiment in a subject by a method or pathway that causes at least partial localization on a desired site.

The administration may be performed by a method known in the art. The administration may be performed directly to a subject by any means, for example, intravenous, intramuscular, oral, transdermal, mucosal, intranasal, intratracheal, or subcutaneous routes. The administration may be administered systemically or locally. The administration may include application to the skin.

The subject may be mammals, such as humans or mammals other than humans (e.g., cows, horses, pigs, dogs, sheep, goats, or cats). The subject may be the one requiring prevention, amelioration, or treatment of a skin condition, such as the one requiring skin barrier strengthening effects or prevention, amelioration, or treatment of skin inflammation.

Regarding the administration, the composition according to an embodiment may be administered at a dosage of about 0.1 mg to 1,000 mg per subject, for example, about 0.1 mg to 500 mg, about 0.1 mg to 100 mg, about 0.1 mg to 50 mg, about 0.1 mg to 25 mg, about 1 mg to 1,000 mg, about 1 mg to 500 mg, about 1 mg to 100 mg, about 1 mg to 50 mg, about 1 mg to 25 mg, about 5 mg to 1,000 mg, about 5 mg to 500 mg, about 5 mg to 100 mg, about 5 mg to 50 mg, about 5 mg to 25 mg, about 10 mg to 1,000 mg, about 10 mg to 500 mg, about 10 mg to 100 mg, about 10 mg to 50 mg, or about 10 mg to 25 mg, per subject. However, the dosage may be variously prescribed depending on factors, such as a formulation method, an administration method, age, weight, gender, and medical conditions of a patient, food, administration time, an administration route, an excretion rate, and response sensitivity, and a person skilled in the art may appropriate adjust the dosage in consideration of these factors. The number of administration may be once a day or at least twice a day within the range of clinically acceptable side effects, and the administration may be performed at a single site or at least two sites, daily or every 2 days to 5 days. The total number of administration days may be 1 day to 30 days per treatment. As needed, the same treatment may be repeated after the a suitable period of time. For animals other than humans, the same dosage per kg as for humans may be used, or for example, a dosage converted from the aforementioned dosage by the volume ratio (e.g., average value) of the organ (e.g., heart, etc.) between a target animal and the human may be used.

The terms or elements mentioned in the 'method for the prevention, amelioration, or treatment' that are the same as those mentioned in the description of the 'strain', 'lysis solution, culture, or extract', 'use, microbial agent, or microbial fertilizer', 'method or plant', or 'use or composition' are understood to be the same as those mentioned in the previous description of the 'strain', 'lysis solution, culture, or extract', 'use, microbial agent, or microbial fertilizer', 'method or plant', or 'use or composition'.

### Advantageous Effects of Invention

The novel *Bacillus megaterium* HyangYak-01 strain according to one aspect exhibits effects of increasing diversity of microbiome species in soil from crop fields or improving physiochemical properties, promoting crop growth or biosynthesis of biologically active substances, and the like, and thus can be applied for various uses, such as improving soil condition, increasing crop cultivation efficiency, or enhancing crop efficacy. In addition, the *Centella asiatica* cultivated after treatment with the novel *Bacillus megaterium* HyangYak-01 strain, or the extract thereof, according one aspect significantly increased the amount of pharmacological substances, demonstrating effects of increasing the expression of factors related to skin barrier strengthening and effects of inhibiting the expression of pro-inflammatory cytokines. As a result, it can be diversely applied for improving skin condition or preventing or treating skin inflammatory diseases.

### Brief Description of the Drawings

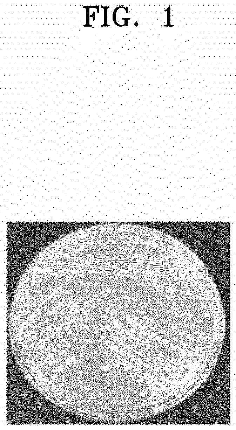

FIG. 1 is a diagram showing results of pure isolation of HyangYak-01 strain colonies.

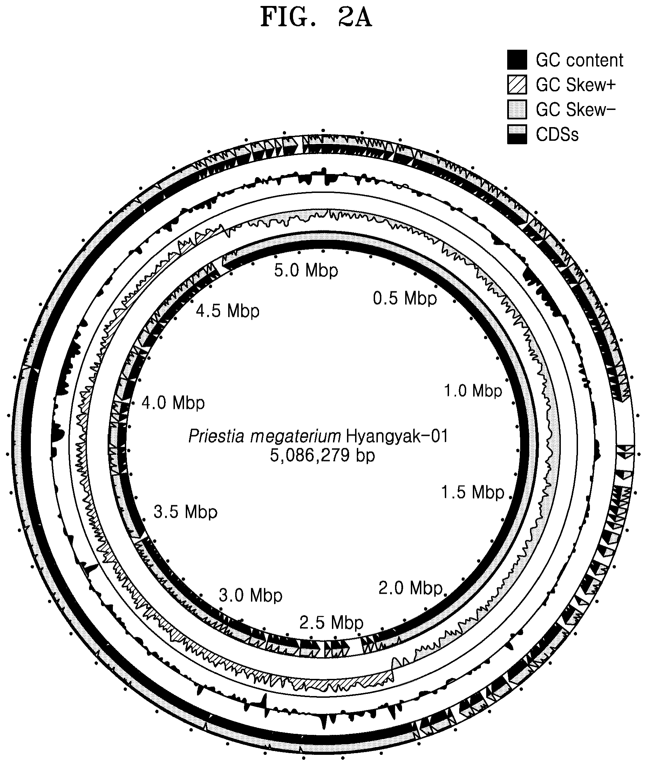

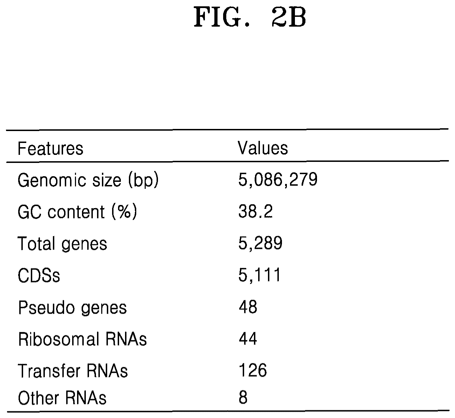

FIGS. 2A and 2B are diagrams showing results of genome sequence analysis of the HyangYak-01 strain.

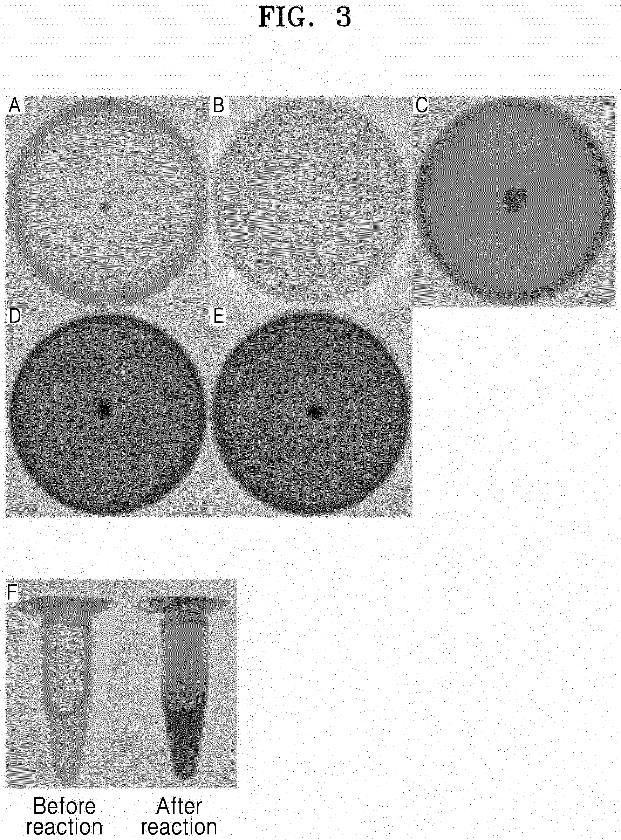

FIG. 3 is a diagram showing results of analyzing the following activities: (A) nitrogen fixation activity of the HyangYak-01 strain; (B) phosphate solubilization activity; (C) urase activity; (D) denitrification activity of nitrite; (E) denitrification activity of nitrate; and production activity of a root growth-promoting hormone.

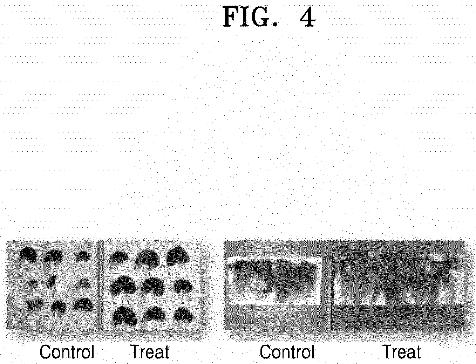

FIG. 4 is a diagram showing results of visual observation of leaves and roots of crops *(Centella asiatica*) that have been treated with the HyangYak-01 strain and cultivated (Control: crops cultivated without treatment with the HyangYak-01 strain; Treat: crops cultivated with treatment with the HyangYak-01 strain).

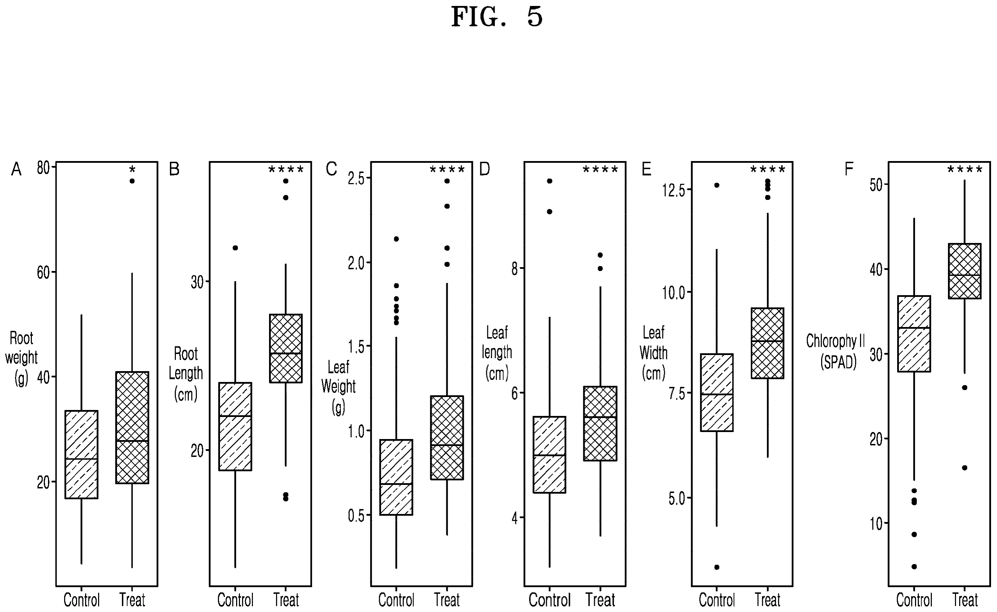

FIG. 5 is a diagram showing results of measuring the growth of crops (*Centella asiatica*) that have been treated with the HyangYak-01 strain and cultivated (Control: crops cultivated without treatment with the HyangYak-01 strain; Treat: crops cultivated with treatment with the HyangYak-01 strain) (A: root weight; B: root length; C: leaf weight; D: leaf length; E: leaf width; F: chlorophyll amount).

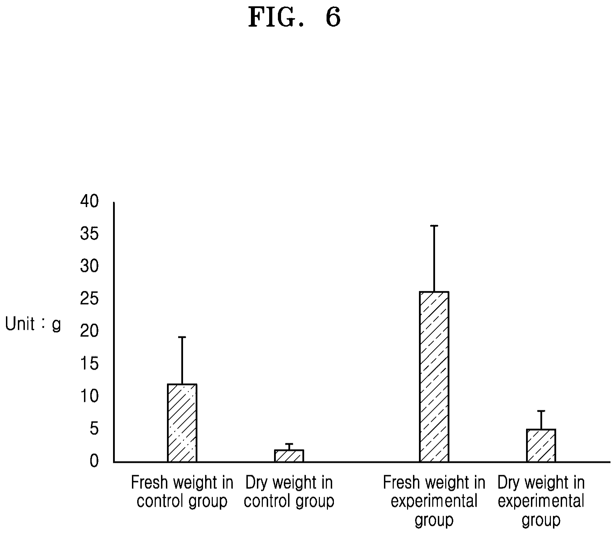

FIG. 6 is a diagram showing results of measuring the fresh weight and dry weight of crops *(Centella asiatica*) that have been treated with the HyangYak-01 strain and cultivated (Control group: crops cultivated without treatment with the HyangYak-01 strain; Experimental group: crops cultivated with treatment with the HyangYak-01 strain).

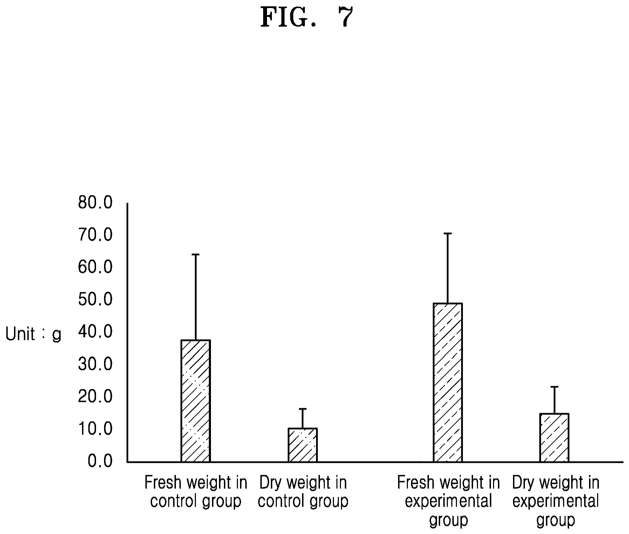

FIG. 7 is a diagram showing results of measuring the fresh weight and dry weight of crops (*Platycodon grandiflorum*) that have been treated with the HyangYak-01 strain and cultivated (Control group: crops cultivated without treatment with the HyangYak-01 strain; Experimental group: crops cultivated with treatment with the HyangYak-01 strain).

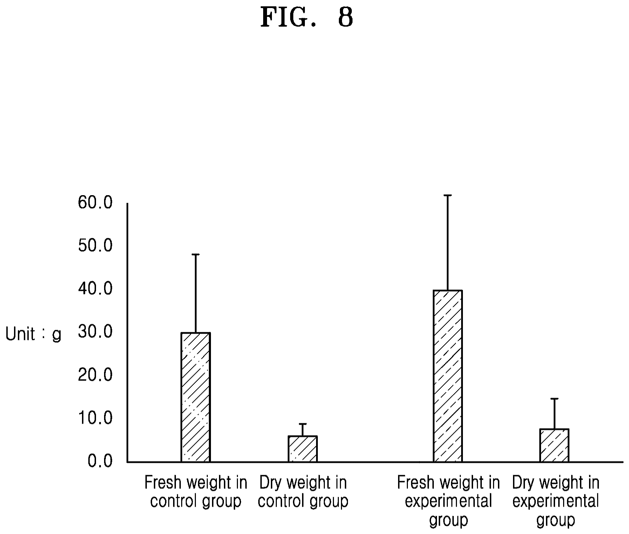

FIG. 8 is a diagram showing results of measuring the fresh weight and dry weight of crops (*Codonopsis lanceolata*) that have been treated with the HyangYak-01 strain and cultivated (Control group: crops cultivated without treatment with the HyangYak-01 strain; Experimental group: crops cultivated with treatment with the HyangYak-01 strain).

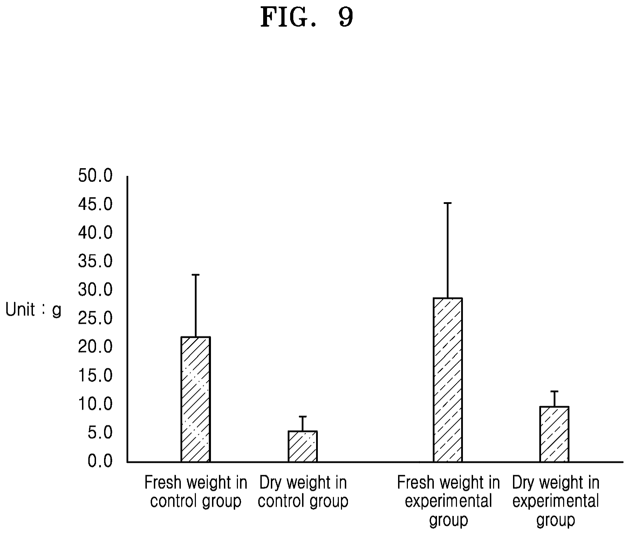

FIG. 9 is a diagram showing results of measuring the fresh weight and dry weight of crops (*Potentilla chinensis*) that have been treated with the HyangYak-01 strain and cultivated (Control group: crops cultivated without treatment with the HyangYak-01 strain; Experimental group: crops cultivated with treatment with the HyangYak-01 strain).

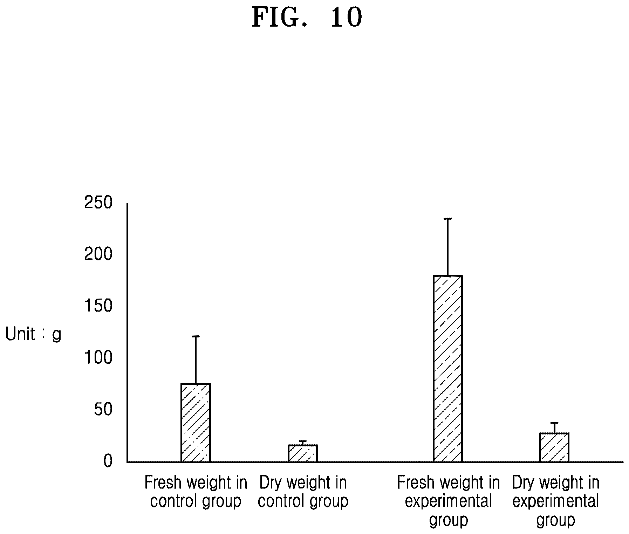

FIG. 10 is a diagram showing results of measuring the fresh weight and dry weight of crops (*Isodon japonicus*) that have been treated with the HyangYak-01 strain and cultivated (Control group: crops cultivated without treatment with the HyangYak-01 strain; Experimental group: crops cultivated with treatment with the HyangYak-01 strain).

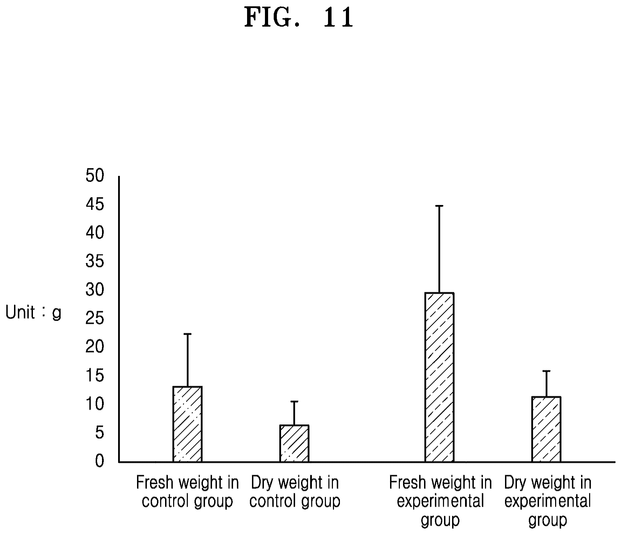

FIG. 11 is a diagram showing results of measuring the fresh weight and dry weight of crops (*Glycyrrhiza uralensis*) that have been treated with the HyangYak-01 strain and cultivated (Control group: crops cultivated without treatment with the HyangYak-01 strain; Experimental group: crops cultivated with treatment with the HyangYak-01 strain).
FIG .12 is a diagram showing results of measuring a root fresh weight of crops *(Centella asiatica*) that have been treated with either the HyangYak-01 strain or the KCTC 3007^{T} strain (Control: crops cultivated without treatment with any strain; KCTC 3007^{T}: crops cultivated with treatment with the KCTC 3007^{T} strain; HyangYak-01: crops cultivated with treatment with the HyangYak-01 strain).
FIG .13 is a diagram showing results of measuring a root dry weight of crops *(Centella asiatica*) that have been treated with either the HyangYak-01 strain or the KCTC 3007^{T} strain (Control: crops cultivated without treatment with any strain; KCTC 3007^{T}: crops cultivated with treatment with the KCTC 3007^{T} strain; HyangYak-01: crops cultivated with treatment with the HyangYak-01 strain).
FIG .14 is a diagram showing results of measuring a leaf fresh weight of crops *(Centella asiatica*) that have been treated with either the HyangYak-01 strain or the KCTC 3007^{T} strain (Control: crops cultivated without treatment with any strain; KCTC 3007^{T}: crops cultivated with treatment with the KCTC 3007^{T} strain; HyangYak-01: crops cultivated with treatment with the HyangYak-01 strain).
FIG .15 is a diagram showing results of measuring a leaf dry weight of crops *(Centella asiatica*) that have been treated with either the HyangYak-01 strain or the KCTC 3007^{T} strain (Control: crops cultivated without treatment with any strain; KCTC 3007^{T}: crops cultivated with treatment with the KCTC 3007^{T} strain; HyangYak-01: crops cultivated with treatment with the HyangYak-01 strain).
FIG .16 is a diagram showing results of measuring the number of leaves of crops *(Centella asiatica*) that have been treated with either the HyangYak-01 strain or the KCTC 3007^{T} strain (Control: crops cultivated without treatment with any strain; KCTC 3007^{T}: crops cultivated with treatment with the KCTC 3007^{T} strain; HyangYak-01: crops cultivated with treatment with the HyangYak-01 strain).

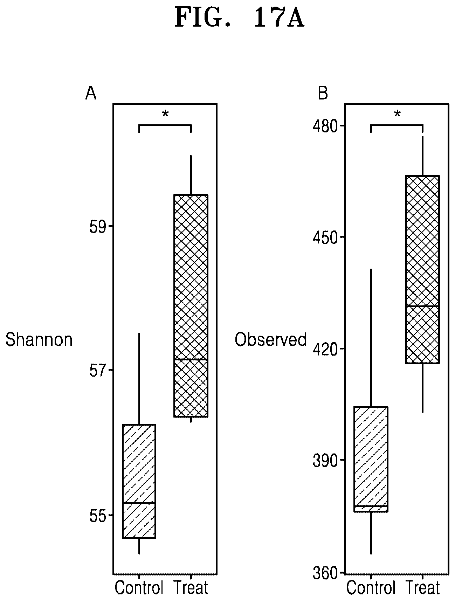

FIG. 17A is a diagram showing results of analyzing changes in the microbiome in soil from crop fields treated with the HyangYak-01 strain (analysis results on indicators of species diversity in the microbiome: (A) Shannon index (A) and (B) Observed OUT).

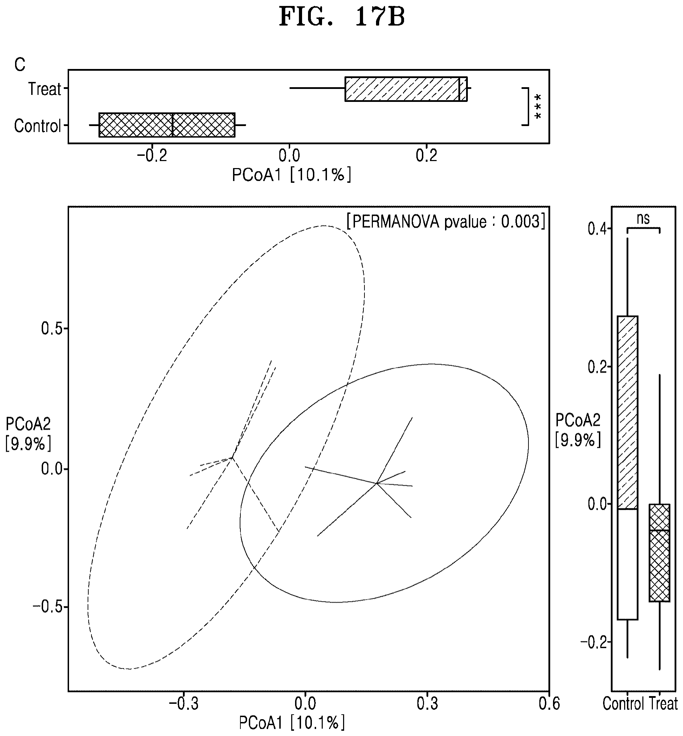

FIG. 17B is a diagram showing results of (C) beta diversity analysis based on PCoA1 and PCoA2 analysis (C) (Control: soil not treated with the HyangYak-01 strain; Treat: soil treated with the HyangYak-01 strain).

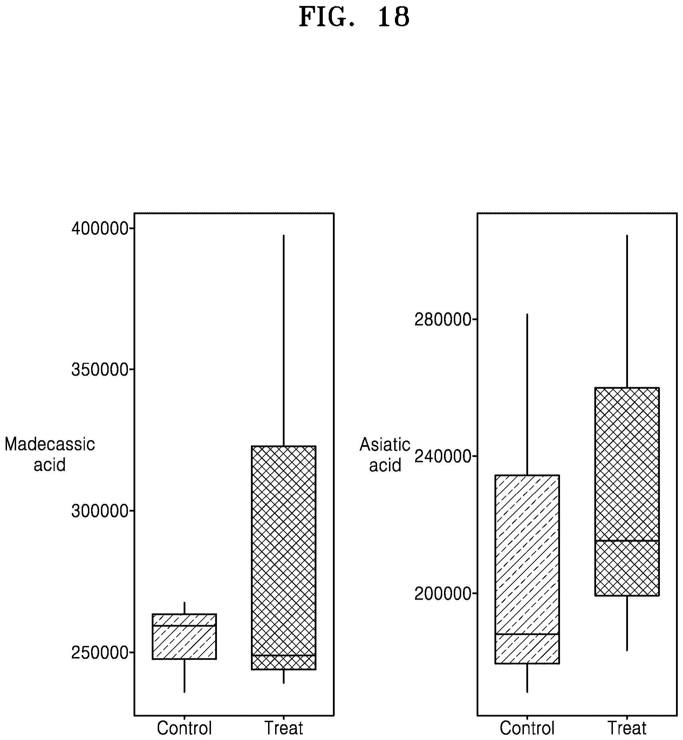

FIG. 18 is a diagram showing LC-TOF/MS results of qualitative analysis of amounts of pharmacological substances (madecassic acid and asiatic acid) in *Centella asiatica* cultivated after treatment with the HyangYak-01 strain (madecassic acid and asiatic acid) (Control: *Centella asiatica* cultivated without treatment with the HyangYak-01 strain; Treat: *Centella asiatica* cultivated after treatment with the HyangYak-01 strain; y-axis: intensity).

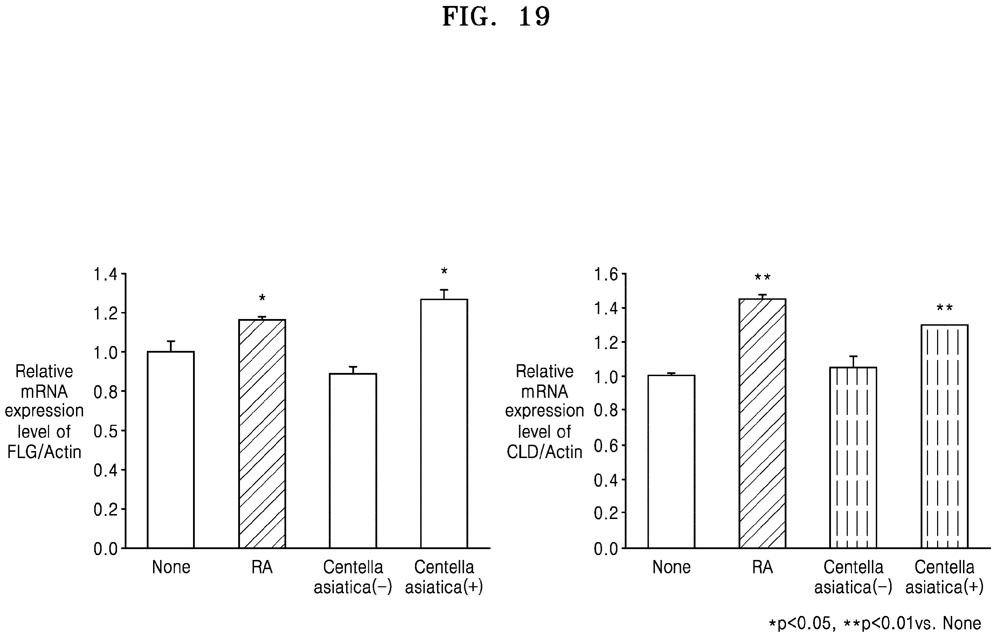

FIG. 19 is a diagram showing results of analyzing effects of a *Centella asiatica* extract, which is prepared by extracting *Centella asiatica* that has been treated with the HyangYak-01 strain and cultivated, on expression of skin barrier strengthening-related factors (FLG and CLD1) (None: group untreated with test substances; RA: group treated with 1 µm of retinoic acid; *Centella asiatica* (-): group treated with an extract of *Centella asiatica* cultivated without treatment with the HyangYak-01 strain; *Centella asiatica*(+): group treated with an extract of *Centella asiatica* cultivated after treatment with the HyangYak-01 strain).

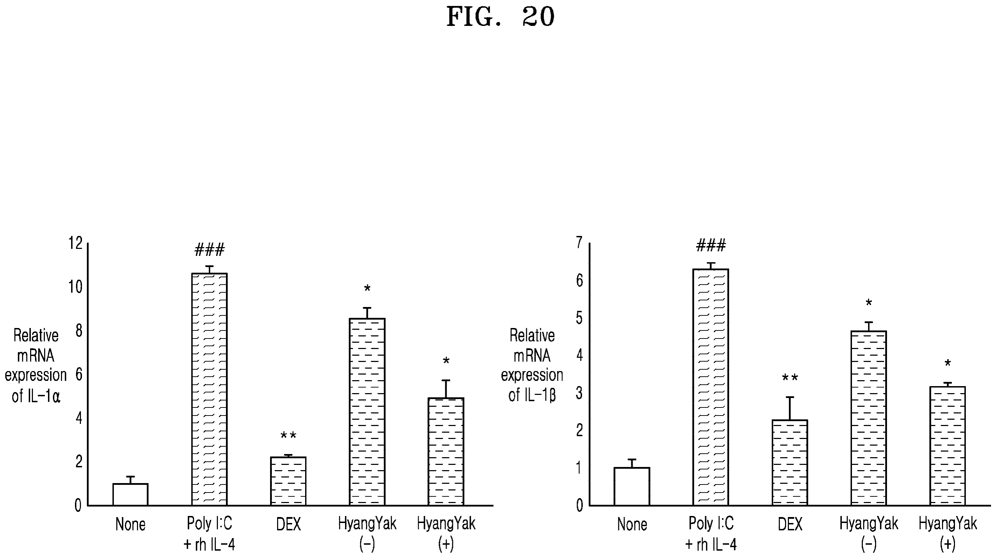

FIG. 20 is a diagram showing results of analyzing effects of a *Centella asiatica* extract, which is prepared by extracting *Centella asiatica* that has been treated with the HyangYak-01 strain and cultivated, on expression of pro-inflammatory cytokines (IL-1α and IL-1β) (None: group of normal cell untreated with test substances; Poly I:C + rh IL-4: group of inflamed cells untreated with test substances; DEX: group of inflamed cells treated with 1 µm of dexamethasone; HyangYak(-): group of inflamed cells treated with an extract of *Centella asiatica* cultivated without treatment with the HyangYak-01 strain; HyangYak(+): group of inflamed cells treated with an extract of *Centella asiatica* cultivated after treatment with the HyangYak-01 strain.

### Best Mode for Carrying out the Invention

### Mode for the Invention

Hereinafter, the present disclosure will be described in detail with reference to Examples below. However, these Examples are for illustrative purposes only, and the scope of the present disclosure is not intended to be limited by these Examples.

### Example 1. Isolation and identification of strain

*A Bacillus megaterium* strain was isolated and identified from rhizosphere soil.

Specifically, soil samples were collected from crops planted in the area of 11-37, Yugugyebong-gil, Yugu-eup, Gongju-si, Chungcheongnam-do. The collected soil samples were subjected to serial dilution with distilled water, and then spread on the Reasoner's 2A (R2A) agar plate and cultured at about 30 °C for about 72 hours. Afterwards, among the bacterial colonies cultured on the agar plate, white colonies were streaked to isolate a single species of bacterial colony, and the results are shown in FIG .1.

Furthermore, the isolated colonies were cultured, and genome gene analysis and 16S rRNA gene sequencing were performed to identify the isolated strain. Specifically, the colonies cultured on the R2A agar medium were sent to Macrogen for 16S rRNA gene sequencing, and the analysis used a primer set of 27F (SEQ ID NO: 2: AGA GTT TGA TCM TGG CTC AG) and 1492R (SEQ ID NO: 3: GGT TAC CTT GTT ACG ACT TC). The analyzed gene information was identified using Ezbiocloud database of CJbioscience (https://www.ezbiocloud.net/).

As a result, the isolated strain was identified as *Bacillus megaterium* including a 16S rRNA gene sequence having a base sequence of SEQ ID NO: 1, and was named HyangYak-01. In addition, the *Bacillus megaterium* HyangYak-01 strain was deposited at the Korean Culture Center of Microorganisms and assigned the Accession No. KCCM13074P. The results of genome gene analysis of the *Bacillus megaterium* HyangYak-01 strain are shown in FIGS. 2A and 2B.

### Experimental Example 1. Analysis of HyangYak-01 strain activity

The following activities of the HyangYak-01 strain were analyzed: production activity of a root growth-promoting hormone (indole-3-acetic acid: IAA); nitrogen fixation activity; phosphate solubilization activity; urea hydrolysis enzyme (urease) activity; and denitrification activity.

Specifically, to confirm the production activity of a root growth-promoting hormone (IAA) of the HyangYak-01 strain, the HyangYak-01 strain was inoculated into the R2A broth (+0.1 % tryptophan), cultured at about 30 °C and about 150 rpm for about 48 to 72 hours, and then centrifuged at about 7,000 rpm for about 20 minutes. The supernatant obtained by the centrifugation was mixed with Salkowski reagent (a mixture of 49 mL of 35 % perchloric acid and 1 mL of 0.5 M ferric chloride) at a ratio of 1:1, and caused a reaction for about 30 minutes under dark conditions, after which the color development was checked. When the reaction solution turns red, the strain may be determined to produce IAA.

In addition, to confirm the nitrogen fixation activity of the HyangYak-01 strain, Jensen's agar containing no nitrogen source was used. About 6 g of sucrose, about 0.2 g of MgSO₄·7H₂O, about 13.9 g of Na₂HPO₄, about 1.7 g of KH₂PO₄, about 2 g of NaCl, about 8 mg of FeCl₃-6H₂O, and about 1 mg of thiamin were dissolved in about 1 L of distilled water, and about 15 g of agar powder was added thereto. The mixture sterilized at about 121 °C for about 15 minutes to prepare Jensen's agar medium. The HyangYak-01 strain was inoculated into the agar medium and cultured at about 30 °C to confirm the nitrogen fixation activity according to the strain growth.

In addition, to confirm the phosphate solubilization of the HyangYak-01 strain, about 5 g of Ca₃(PO₄)₂, about 10 g of sucrose, about 0.5 g of yeast extract, about 0.27 g of NH₄NO₃, about 0.2 g of KCI, about 0.1 g of MgSO₄·7H₂O, about 0.001 g of MnSO₄, and about 0.001 g of FeSO₄·7H₂O were added to about 1 L of distilled water, after which a pH of the mixed solution was adjusted to 7.0. Afterwards, about 15 g of agar power was added thereto, followed by sterilization at about 121 °C for about 15 minutes, to prepare an agar medium. The HyangYak-01 strain was inoculated into the agar medium and cultured at about 30 °C to confirm the phosphate solubilization activity depending on whether a clear zone was formed.

In addition, to confirm the urase activity of the HyangYak-01 strain, urea agar was used. About 20 g of urea, about 1 g of dextrose, about 1 g of peptone, about 5 g of NaCl, about 2 g of KH₂PO₄, and about 122 mg of phenol were dissolved in about 1 L of distilled water, and about 15 g of agar powder was added thereto, followed by sterilization at about 121 °C for about 15 minutes, to prepare a urea agar medium. The HyangYak-01 strain was inoculated into the agar medium and cultured at about 30 °C, and then, the enzymatic activity of urase was confirmed according to discoloration of the agar medium from yellow to pink.

In addition, to confirm the denitrification activity (nitrate: NO₃-) of the HyangYak-01 strain, about 1.0 g of NaNO₃, about 1.0 g of asparagine, about 5 ml of bromothymol blue solution (containing about 1 g of bromothymol blue, about 50 ml of ethanol, and about 50 ml of distilled water), about 8.5 g of sodium citrate, about 1.0 g of MgSO₄·7H₂O, about 0.05 g of FeCl₃·6H₂O, about 1.0 g of KH₂PO₄, and about 0.2 g of CaCl₂·2H₂O were added to about 1 L of distilled water, after which the pH was adjusted to 7.0. Then, about 15 g of agar powder was added thereto, followed by sterilization at about 121 °C for about 15 minutes, to prepare an agar medium. The HyangYak-01 strain was inoculated into the agar medium and cultured at about 30 °C to confirm the denitrification activity depending on whether a clear zone was formed in a blue medium upon the strain growth.

In addition, to confirm the denitrification activity (nitrite: NO₂-) of the HyangYak-01 strain, about 0.006 g of NaNO₂, about 0.03 g of FeSO₄·7H₂O, about 1.0 g of K₂HPO₄, about 0.3 g of CaCl₂·2H₂O, about 0.3 g of NaCl, about 1.0 g of NaHCO₃, and about 0.1 g of MgSO₂·7H₂O were distilled water in about 1.0 L of distilled water, and about 15 g of agar powder was added thereto, followed by fertilization at about 121 °C for about 15 minutes, to prepare an agar medium. The HyangYak-01 strain was inoculated into the agar medium and cultured at about 30 °C. Following the culturing, the Griess test (i.e., a test for determining whether a clear zone was formed around the strain by dispensing a solution prepared by mixing a first solution with a second solution onto an agar plate where the strain was cultured, about 0.5 g of sulfanilic acid was dissolved in about 70 ml of boiling distilled water, cooled, and mixed with about 30 ml of acetic acid, and the mixed solution was titrated to about 100 ml to prepare the first solution; and about 0.5 g of 1-naphthylamine sol. : 1-naphthylamine was dissolved in about 30 ml of acetic acid, and about 70 ml of distilled water was added thereto to prepare the second solution) was performed to confirm the denitrification activity depending on whether a clear zone was formed around the strain.

As a result, as shown in FIG. 3, it was confirmed that the solution obtained by reacting the supernatant of the culture broth of the HyangYak-01 strain with the Salkowski reagent turned red, indicating that the strain exhibits the production activity of a root growth-promoting hormone. In addition, it was confirmed that the HyangYak-01 strain could also grow in the Jensen's agar medium containing no nitrogen source, indicating that the strain exhibits the nitrogen fixation activity. In addition, it was confirmed that, when the HyangYak-01 strain was inoculated into the Ca₃(PO₄)₂-containing agar medium and cultured, a clear zone was formed around the strain as the strain grew, indicating that the strain exhibits the phosphate solubilization activity. In addition, it was confirmed that, when the HyangYak-01 strain was inoculated into the urea agar medium, the urea agar medium changed color from yellow to pink as the strain grew, indicating that the strain exhibits the urase enzyme activity. In addition, as a results of confirming the denitrification activity, it was confirmed that a clear zone was formed around the HyangYak-01 strain as the strain grew on the agar medium, indicating that the strain exhibits the denitrification activity.

### Experimental Example 2. Gene analysis of HyangYak-01 strain involved in crop growth-promoting activity

Genes of the HyangYak-01 strain involved in crop growth-promoting activity were analyzed.

Specifically, to confirm genes involved in crop growth-promoting activity, DNA of the HyangYak-01 strain cultured in the R2A broth was extracted using a Wizard Genomic DNA Purification Kit (Promega). The extracted DNA was checked for quality using NanoDrop, and the nucleic acid sequencing was performed thereon using Oxford Nanopore (Oxford Nanopore Technologies, Oxford, UK) and MGI DNBSEQ-G400 (MGI Tech, Shenzhen, China). The analyzed nucleic acid sequences were assembled using Flye de novo assemble (version : 2.9.1) and analyzed. Using the prokka tool, genes related to the activities evaluated in Experimental Example 1 were identified from the genetic information obtained through the whole genome sequencing.

As a result, it was confirmed that, as shown in Table 1, the HyangYak-01 strain had genes related to the production of root growth-promoting hormone, nitrogen fixation, phosphate solubilization, urase enzyme, and denitrification, which are involved in the crop growth-promoting activity. Accordingly, it was confirmed that the HyangYak-01 strain exhibits the production activity of a root growth-promoting hormone, nitrogen fixation activity, phosphate solubilization activity, urase enzyme activity, and denitrification activity, and accordingly promotes the crop growth.

**Table 1**

| **Gene name** | **Explanation** | **Source** |
|---|---|---|
| gatA | IAA biosynthesis pathways | Indole-3-acetic acid biosynthesis pathways in the plant-beneficial bacterium Arthrobacter pascens ZZ21 |
| puuPER | Urease | Urease |
| aldH | IAA biosynthesis pathways | Indole-3-acetic acid biosynthesis pathways in the plant-beneficial bacterium Arthrobacter pascens ZZ21 |
| ytnP | Quorum-quenching lactonase YtnP | Quorum-quenching lactonase YtnP |
| phoP | Alkaline phosphatase genes | Genome mining of three plant growth-promoting Bacillus species from maize rhizosphere |
| phoR | Alkaline phosphatase genes | Genome mining of three plant growth-promoting Bacillus species from maize rhizosphere |
| phoH | Alkaline phosphatase genes | Genome mining of three plant growth-promoting bacillus species from maize rhizosphere |
| nifS | - | Cysteine desulfurase |
| narT | Nitrate transproter gene | Nitrate transproter gene |
| nirCQ | Nitrite transporter gene | Nitrite transporter gene |
| norRMG | - | Anaerobic nitric oxide reductase transcription regulator NorR |
| ureGFECBDARI | Urease gene cluster | Bacterial urease and its role in long-lasting human diseases |
| ureDGFECBA | - | - |

Experimental Example 3. Confirmation of crop growth-promoting effect of HyangYak-01 strain

### 3-1. Confirmation of growth-promoting effect of HyangYak-01 strain on Centella asiatica

The growth changes of crops cultivated after treatment with the HyangYak-01 strain (specifically, changes in root length, root weight, leaf length, leaf weight, leaf width, and chlorophyll amount) were analyzed to confirm the growth-promoting effect of the HyangYak-01 strain on crops.

Specifically, *Centella asiatica* seedlings were planted in soil near 11-37, Yugugyebong-gil, Yugu-eup, Gongju-si, Chungcheongnam-do. Afterwards, a culture broth obtained by culturing the HyangYak-01 strain in a R2A medium at about 30 °C and about 150 rpm for about 48 to 72 hours was centrifuged at about 7,000 rpm for about 20 minutes, after which the supernatant was removed and only the bacterial cells were obtained. The bacterial cells were diluted 100 times and applied to the soil used for planting the *Centella asiatica* seedlings, and a group treated only with water instead of the strain was set as a control group. Afterwards, the soil where the *Centella asiatica* seedlings were planted was watered at intervals of about 1 and 2 weeks, and open field cultivation was carried out for about 3 months. Then, the harvested *Centella asiatica* was washed, drained, and measured for the root length, leaf length, and leaf width. After drying, the root weight and leaf weight were measured using a scale. The chlorophyll was measured using a SPAD-502 Plus measuring device.

As a result, as shown in FIGS. 4 and 5, it was confirmed that, in the case of the *Centella asiatica* cultivated with the treatment with the HyangYak-01 strain, the root length, root weight, leaf length, leaf weight, leaf width, and chlorophyll amount were significantly increased compared the untreated control group. Accordingly, it was confirmed that the HyangYak-01 strain exhibited an effect of significantly promoting the growth of crops (e.g., *Centella asiatica).*

### 3-2. Confirmation of growth-promoting effect of HyangYak-01 strain on various crops

To confirm whether the HyangYak-01 strain exhibits an effect on crops other than the *Centella asiatica,* the growth changes (specifically, the root fresh weight and root dry weight) of various crops cultivated with treatment with the HyangYak-01 strain were analyzed, and the growth-promoting effect of the HyangYak-01 strain on crops was confirmed.

Specifically, seedlings of *Angelica giga, Platycodon grandiflorum, Codonopsis lanceolata, Potentilla chinensis, Isodon japonicus,* and *Glycyrrhiza uralensis* were planted in soil near 11-37, Yugugyebong-gil, Yugu-eup, Gongju-si, Chungcheongnam-do. Afterwards, a culture broth obtained by culturing the HyangYak-01 strain in a R2A medium at about 30 °C and about 150 rpm for about 48 to 72 hours was centrifuged at about 7,000 rpm for about 20 minutes, after which the supernatant was removed to obtain only the bacterial cells. The bacterial cells were diluted 100 times and applied to the soil used for planting the crops seedlings, and a group treated only with water instead of the strain was set as a control group. Afterwards, the soil where the crop seedlings were planted was watered at intervals of about 1 and 2 weeks, and open field cultivation was carried out for about 3 months. Afterwards, the harvested crops were each washed, drained, and measured for the root fresh weight. After drying, the root dry weight was measured.

As a result, as shown in FIGS. 6 to 11, it was confirmed that, all of the *Angelica giga, Platycodon grandiflorum, Codonopsis lanceolata, Potentilla chinensis, Isodon japonicus,* and *Glycyrrhiza uralensis* cultivated with the treatment with the HyangYak-01 strain showed an increase in the root fresh weight and root dry weight, compared to the untreated control group. Accordingly, it was confirmed that the HyangYak-01 strain exhibited an effect of enhancing the growth of not only the *Centella asiatica* but also various crops.

### 3-3. Comparative evaluation of crop growth-promoting effects between HyangYak-01 strain and reference strain

To compare and evaluate the crop growth-promoting effects between the HyangYak-01 strain and a reference strain, a reference strain of *Priestia megaterium, Priestia megaterium* KCTC 3007^{T}, was obtained from the Biological Resource Center of the Korea Research Institute of Bioscience and Biotechnology, and used. Then, the growth changes (specifically, differences in root fresh weight, root dry weight, leaf fresh weight, leaf dry weight, and number of leaves) in crops cultivated with treatment with each of the HyangYak-01 strain and the KCTC 3007^{T} strain were compared and analyzed.

Specifically, after transplanting the *Centella asiatica* seedlings into pots, the HyangYak-01 strain and the KCTC 3007^{T} strain were each cultured in a R2A medium at about 30 °C and about 150 rpm for about 48 to 72 hours. Afterwards, the two types of obtained culture broth were each centrifuged at about 7,000 rpm for about 20 minutes, and the supernatant was removed to obtain only the two types of bacterial cells. Each of the two types of the bacterial cells was diluted 100 times and applied to the transplanted *Centella asiatica* seedlings. In addition, a group treated only with sterile distilled water instead of with bacterial cells was set as a control group. Afterwards, the transplanted *Centella asiatica* seedlings were treated with the HyangYak-01 bacterial cells, the KCTC 3007^{T} bacterial cells, and sterile distilled water, each three times at weekly intervals, and cultivation was carried out for one month in a steady temperature and humidity room at about 26 °C and a humidity of about 40 to 60 %. Afterwards, the harvested *Centella asiatica* was washed, drained, and measured for the root fresh weight and leaf fresh weight. After drying, the root dry weight and leaf dry weight were measured. Also, the number of leaves was counted.

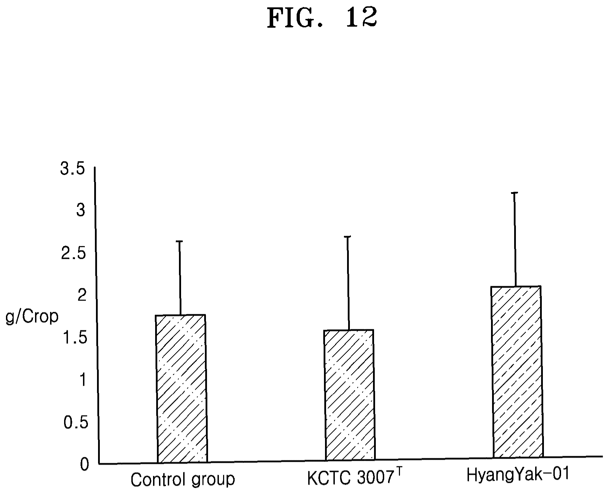

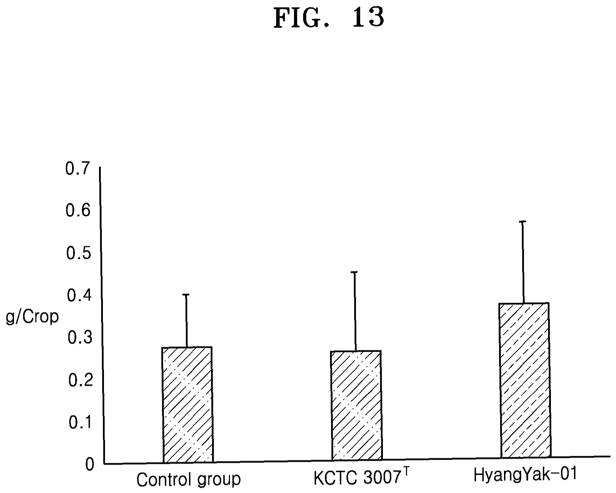

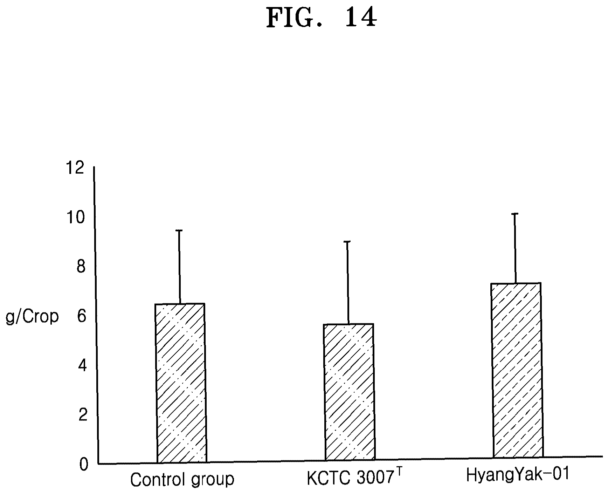

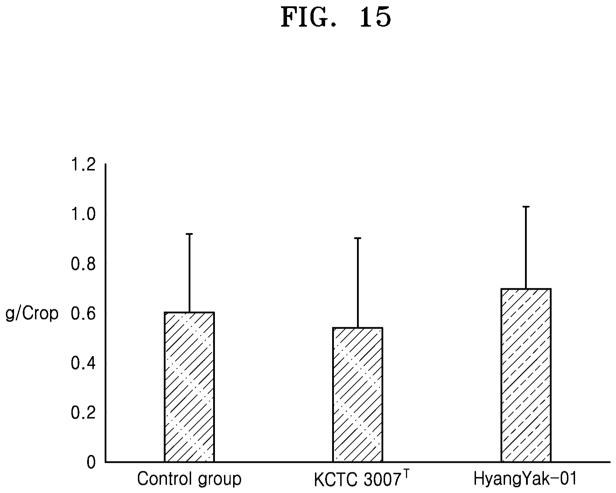

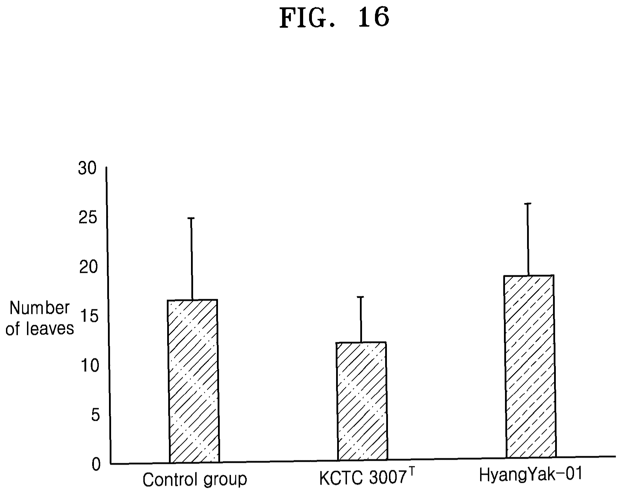

As a result, as shown in FIGS. 12 to 16, it was confirmed that, in the case of *Centella asiatica* cultivated with treatment with the HyangYak-01 strain, the root fresh weight, root dry weight, leaf fresh weight, leaf dry weight, and number of leaves were all increased compared to the group treated with sterile distilled water (control) and the group treated with the KCTC 3007^{T} strain. Accordingly, it was confirmed that there were differences in the crop growth-promoting effects even among different strains belonging to the same species, i.e., different strains belonging to *Priestia megaterium,* and that the HyangYak-01 strain showed excellent crop growth-promoting effects compared to other strains *e.g., reference strain) belonging to *Priestia megaterium.* In other words, it could be understood that, as a result of including 16S rRNA having the base sequence of SEQ ID NO: 1, the HyangYak-01 strain had differences in base sequences (i.e., genetic differences) from other strains belonging to *Priestia megaterium.*

### Experimental Example 4. Confirmation of changes in microbiomes in soil from crop fields by treatment with HyangYak-01 strain

Changes in microbiomes in soil from crop fields treated with the HyangYak-01 strain were analyzed.

Specifically, at the time of cultivation of the *Centella asiatica* in Experimental Example 3-1, soil samples were collected from the experimental group treated with the HyangYak-01 strain and the control group not treated with the HyangYak-01 strain, and then, DNA was extracted from the soil samples using the DNeasy PowerSoil Pro Kits (QIAGEN). Using the extracted DNA, an amplicon library was prepared to analyze the bacterial community, which was performed through sequencing using Miseq equipment. Then, the changes in microbiome were analyzing using Qiime 2 for the sequenced data. Specifically, the changes in diversity of soil microbiome species were analyzed by deriving Shannon index and Observed OUT values, which represent the species diversity. Then, according to the results obtained by analyzing PCoA1 and PCoA2, grouping of the experimental group and the control group was performed, followed by beta diversity analysis for analyzing the changes in soil microbiome clusters.

As a result, as shown in FIGS. 17A and 17B, it was confirmed that the Shannon index and Observed OUT values analyzed from the samples of soil from crop fields treated with the HyangYak-01 strain were significantly higher than those analyzed from the samples of the control group not treated with the HyangYak-01 strain. Accordingly, it was confirmed that the soil treated with the HyangYak-01 strain showed a significant increase in the diversity of microbiome species compared to the soil not treated with the HyangYak-01 strain. In addition, the beta diversity analysis results showed that, depending on the treatment with the HyangYak-01 strain, the soil microbiomes diverged, resulting in significant changes in the microbial communities.

According to the present Experimental Example, it was confirmed that, when the soil was treated with the HyangYak-01 strain, the soil may become healthier for growing crops due to changes in the microbial communities, such as an increase in diversity of microbiome species, and as a result, the growth of crops cultivated in the soil was promoted.

### Experimental Example 5. Confirmation of effects of improving physiochemical properties of soil from crop fields by treatment with HyangYak-01 strain

Changes in physiochemical properties in soil from crop fields treated with the HyangYak-01 strain were analyzed.

Specifically, Specifically, at the time of harvesting the *Centella asiatica* after cultivation in Experimental Example 3-1, about 1 kilogram (kg) of soil was sampled from each of the experimental group treated with the HyangYak-01 strain and the control group untreated with the HyangYak-01 strain (only treated with water instead of the strain), and the obtained soil samples (three samples obtained for each group) were analyzed for physiochemical properties. Specifically, the soil samples were prepared by naturally drying in the shade for 3 days and sieving through about 2 mm sieve. Then, for the prepared soil samples, the pH, electrical conductivity (EC), amount of effective phosphorus, amount of organic matters, and amount of (exchangeable) cations (potassium ions, calcium ions, and magnesium ions) were measured according to the soil chemical analysis method (NIAST, 2010). The pH and EC were measured by extracting soil solution using a 1:5 method. The amount of organic matters was measured by absorbance at 610 nm using a Walkley-Black method, and the amount of effective phosphorus was quantified by colorimetry at 660 nm using a molybdenum blue method. The amounts of the organic matters and effective phosphorus were measured using a UV-vis spectrometer (UV-2401PC, Shimadzu corporation, Japan), and for the amount of cations, the soil sample was extracted using a solution of 1 N NH₄OAc (pH 7.0) and analyzed by ICP (Intergra XL, GBC, Australia). Furthermore, initial soil samples that had not been treated prior to the cultivation of the *Centella asiatica* were also set as a control group, and the same analysis as described above was performed thereon.

As a result, as shown in Table 2, it was confirmed that the soil pH increased after crop cultivation compared to the initial soil, and that the pH increase level in the soil where crops were cultivated after treatment with the HyangYak-01 strain was lower than that in the soil of the control group. In addition, it was confirmed that the amount of organic matters in the soil after crop cultivation decreased compared to the initial soil, and that the amount of organic matters further decreased in the soil where crops were cultivated after treatment with the HyangYak-01 strain compared to that in the soil of the control group. In addition, it was confirmed that the amount of effective phosphorus in the soil after crop cultivation increased compared to the initial soil, and that the increase level of effective phosphorus in the soil where crops were cultivated after treatment with the HyangYak-01 strain was lower than that in the soil of the control group, but was within normal levels. In addition, it was confirmed that the amount of exchangeable potassium ions in the soil after crop cultivation showed no significant changes in the soil where crops were cultivated after treatment with the HyangYak-01 strain compared to the initial soil, whereas, compared to the initial soil, the amounts of exchangeable calcium ions and exchangeable magnesium ions in the soil after crop cultivation further increased in the soil where crops were cultivated after treatment with the HyangYak-01 strain compared to the soil of the control group. In addition, it was confirmed that the EC increased slightly in the soil where crops were cultivated after treatment with the HyangYak-01 strain compared to the initial soil, but was within normal levels.

Through the present Experimental Example, it was found that the soil pH was closely related to nutrient absorption, and crops generally grew best in slightly acidic (pH 6.0 to 6.5) or mildly acidic (pH 6.5 to 7.0) soil. In the case of the HyangYak-01 strain, the pH of soil from crop fields was maintained at slightly acidic or mildly acidic levels, improving the soil condition healthier. In addition, it was confirmed that the HyangYak-01 strain played a major role in transforming organic matters to be available by crops through decomposition of the organic matters in the soil from crop fields, thereby improving the soil condition more suitable for crop cultivation. In addition, the cation substitution capacity (total amount of exchangeable cations) of soil is regarded as an indicator of soil fertility and is involved in improving soil buffering capacity and retention and supply of nutrients, and soil with high cation substitution capacity is known to be fertile because it includes more inorganic nutrients necessary for the growth of crops. In this regard, it was confirmed that the HyangYak-01 strain could improve soil fertility by increasing exchangeable cations such as calcium ions and magnesium ions. In addition, it was confirmed that the HyangYak-01 strain maintained the EC of soil at normal levels without any significant impact, and also maintained the amount of effective phosphorus at normal levels. Overall, when soil was treated with the HyangYak-01 strain, the physiochemical properties of the soil were improved (maintaining the soil pH at slightly acidic or mildly acidic levels; promoting decomposition of organic matters in soil; increasing the amount of exchangeable cations in soil; and maintaining the EC of soil and amount of effective phosphorus at normal levels), making the soil healthier for crop cultivation, and as a result, it was confirmed that the growth of crops cultivated in the soil could be promoted.

**Table 2**

| **Sample** | **pH** | **Organic** | **Effective** | **Exchang eable** | **Excha ngeable** | **Exchang eable** | **Electrical** |
|---|---|---|---|---|---|---|---|
| | | **matters** | **phosphate** | **potassium** | **calcium** | **magnesium** | **conductivity** |
| **Initial soil** | 4.5 | 63.8 | 42.83 | 0.25 | 1.37 | 1.32 | 1 |
| | 4.5 | 63.8 | 42.8 | 0.26 | 1.42 | 1.34 | 1 |
| | 4.52 | 64.01 | 42 | 0.24 | 1.45 | 1.37 | 1 |
| **HyangYak-01-untreated soil** | 7.9 | 19.4 | 520.15 | 0.37 | 11.02 | 3.83 | 0.4 |
| | 7.91 | 19.42 | 521 | 0.37 | 11 | 3.83 | 0.4 |
| | 7.92 | 19.54 | 532.12 | 0.41 | 11.02 | 4.01 | 0.4 |
| **HyangYak-01-treated soil** | 6.9 | 16.5 | 450.28 | 0.26 | 11.87 | 4.13 | 1.3 |
| | 6.91 | 16.54 | 450.31 | 0.25 | 11.81 | 4.15 | 1.3 |
| | 6.95 | 16.52 | 450 | 0.27 | 11.82 | 4.21 | 1.3 |

### Experimental Example 6. Confirmation of changes in amount of pharmacological substances in Centella asiatica cultivated after treatment with HyangYak-01 strain

Changes in the amounts of pharmacological substances (specifically, madecassic acid and asiatic acid) of *Centella asiatica* cultivated after treatment with the HyangYak-01 strain were analyzed.

Specifically, leaves were separated from the *Centella asiatica* cultivated after treatment with the HyangYak-01 strain obtained from Experimental Example 3-1, and the separated leaves were subjected to extraction using about 70 to 100 % ethanol for about 2 to 48 hours. Afterwards, the extract thus obtained was filtered using Whatman paper to remove impurities, and the filtrate was concentrated using a speedvac at about 45 °C and about 5 vacuum pressure for about 3 hours. The concentrated substance was then dissolved in methanol and filtered through a 0.22 µm filter. The filtered sample was then qualitatively analyzed for the amounts of madecassic acid and asiatic acid using LC-TOF/MS. The same analysis was performed on the leaves of the *Centella asiatica,* as a control group, cultivated without treatment with the HyangYak-01 strain.

As a result, as shown in FIG. 18, the *Centella asiatica* cultivated after treatment with the HyangYak-01 strain showed a significant increase in the amounts of madecassic acid and asiatic acid, compared to the untreated control group. Accordingly, it was confirmed that the HyangYak-01 strain could promote the biosynthesis of biologically active substances, such as pharmacological substances, of crops (e.g., *Centella asiatica*)*.*

### Experimental Example 7. Confirmation of efficacy of Centella asiatica cultivated after treatment with HyangYak-01 strain on improvement of skin condition

The efficacy of the *Centella asiatica* cultivated after treatment with the HyangYak-01 strain on improvement of a skin condition (specifically, efficacy of skin barrier strengthening) was analyzed.

Specifically, leaves were separated from the *Centella asiatica* cultivated after treatment with the HyangYak-01 strain obtained from Experimental Example 3-1, and the separated leaves were subjected to extraction using about 70 % ethanol at room temperature for about 48 to 72 hours to obtain an extraction solution. Subsequently, the extraction solution was concentrated using a vacuum evaporator to obtain the *Centella asiatica* extract. Then, the efficacy of the *Centella asiatica* extract on the improvement of a skin condition was confirmed. Specifically, HaCaT cells, a human keratinocyte cell line, were cultured in a Dulbecco's modified Eagle's medium (DMEM) (Gibco 1210-0038) supplemented with 10 % fetal bovine serum, and the culturing was performed at about 37 °C in an about 5 % CO₂ incubator. The cultured cells were treated with the *Centella asiatica* extract at a concentration of about 1 % (w/w), and then additionally cultured for about 24 hours. Subsequently, the relative expression levels of β-actin with respect to each of filaggrin (FLG) and claudin-1 (CLD1), which are factors related to skin barrier strengthening, were analyzed in the cultured cells. Specifically, RNA was isolated from the cultured cells using trizol (RNA iso, DAKARA, Japan), and the RNA was quantified at 260 nm using a nanodrop. Then, cDNA was synthesized in an amplifier (C1000 Thermal Cycler, Bio-Rad, USA) using 2 µg of each RNA. Real-time polymerase chain reaction (PCR) was performed using a real-time PCR machine (Step One Plus, Applied Biosystems, USA) using the synthesized cDNA as a template, together with primers and cDNA for the target genes, i.e., FLG and CLD1 genes. The expression levels of the FLG and CLD1 genes were finally analyzed by correction with respect to the β-actin gene. The same analysis was performed on a group treated with the *Centella asiatica* extract (extraction method was as described above) that has been cultivated without treatment with the HyangYak-01 strain as a control group, a retinoic acid-treated group (treated at a concentration of about 1 µm), and a group that has not been treated with any test substance.

As a result, as shown in FIG. 19, it was confirmed that the *Centella asiatica* extract prepared by extracting the *Centella asiatica* that has been cultivated after treatment with the HyangYak-01 strain showed a significant increase in the expression of factors related to skin barrier strengthening, such as FLG and CLD1, compared to the extract of *Centella asiatica* that has been cultivated without treatment with the HyangYak-01 strain. Accordingly, it was confirmed that the *Centella asiatica* cultivated after treatment with the HyangYak-01 strain, and the extract thereof exhibited excellent efficacy on skin barrier strengthening, suggesting the potential for use in improving a skin condition.

### Experimental Example 8. Confirmation of anti-inflammatory efficacy of Centella asiatica cultivated after treatment with HyangYak-01 strain

Anti-inflammatory efficacy of the *Centella asiatica* cultivated after treatment with the HyangYak-01 strain was analyzed.

Specifically, leaves were separated from the *Centella asiatica* cultivated after treatment with the HyangYak-01 strain obtained from Experimental Example 3-1, and the separated leaves were subjected to extraction using about 70 % ethanol at room temperature for about 48 to 72 hours to obtain an extraction solution. Subsequently, the extraction solution was concentrated using a vacuum evaporator to obtain the *Centella asiatica* extract. Then, the anti-inflammatory efficacy of the *Centella asiatica* extract on the skin was confirmed. Specifically, HaCaT cells, a human keratinocyte cell line, were cultured in a Dulbecco's modified Eagle's medium (DMEM) (Gibco 1210-0038) supplemented with 10 % fetal bovine serum, and the culturing was performed at about 37 °C in an about 5 % CO₂ incubator. During the culturing, the medium was replaced every 3 to 4 days, and when the cells proliferated excessively, subculturing was performed. Subsequently, the cultured cells were seeded into each well at a concentration of about 5x10⁵/well and cultured for about 24 hours. Following the culturing, the cultured cells were washed with phosphate-buffered saline (PBS), and the washed cells were seeded into each well containing a FBS-free DMEM medium, Poly I:C and IL-4 were added at concentrations of about 10 *µg*/^{mℓ} and about 10 ng/^{mℓ}, respectively, to induce inflammation in the cells. Afterwards, the inflammation-induced cells were treated with the *Centella asiatica* extract at a concentration of about 1 % (w/w) and additionally cultured for about 4 hours. Subsequently, the expression levels of pro-inflammatory cytokines, such as IL-1α and IL-1β, were analyzed in the cultured cells. Specifically, RNA was isolated from the cultured cells using trizol (RNA iso, DAKARA, Japan), and the RNA was quantified at 260 nm using a nanodrop. Then, cDNA was synthesized in an amplifier (C1000 Thermal Cycler, Bio-Rad, USA) using 2 µg of each RNA. PCR was performed using a real-time PCR machine (Step One Plus, Applied Biosystems, USA) using the synthesized cDNA as a template, together with primers and cDNA for the target genes, i.e., IL-1α and IL-1β genes, to analyze the expression levels of IL-1α and IL-1β. For the inflammation-induced cells, the same analysis was performed on: the *Centella asiatica* extract (extraction method was as described above), as a control group, cultivated without treatment with the HyangYak-01 strain or a group treated with dexamethasone (treated at a concentration of about 1 µm) or a group that was not treated with the test substance at all; and a group of normal cells without treatment with the test substance at all.

As a result, as shown in FIG. 20, it was confirmed that the *Centella asiatica* extract prepared by extracting the *Centella asiatica* cultivated after treatment with the HyangYak-01 strain showed significant inhibition of the expression of pro-inflammatory cytokines, such as interleukin-1 alpha (IL-1α) and interleukin-1 beta (IL-1β), in the inflammation-induced cells compared to the *Centella asiatica* extract cultivated without treatment with the HyangYak-01 strain. Accordingly, it was confirmed that the *Centella asiatica* cultivated after treatment with the HyangYak-01 strain, or the extract thereof exhibited excellent anti-inflammatory efficacy (i.e., inflammation-inhibiting efficacy), suggesting usability for the prevention, amelioration, or treatment of skin inflammatory diseases (or inflammation with skin inflammatory diseases).

In summary, it was confirmed that the *Bacillus megaterium* HyangYak-01 strain scientifically increased the diversity of microbiome species in the soil from crop fields, improved the physiochemical properties of the soil, and significantly promoted the growth of crops. In particular, it was confirmed that the amount of pharmacological substances significantly increased in the *Centella asiatica* cultivated after treatment with the strain, and as a result, the biosynthesis of pharmacological substances of crops (e.g., *Centella asiatica*) could be promoted by the strain.

Furthermore, the *Centella asiatica* extract prepared by extracting the *Centella asiatica* cultivated after treatment with the strain significantly increased the expression of factors related to skin barrier strengthening and significantly inhibited the expression of pro-inflammatory cytokines.

Therefore, it was confirmed that the *Bacillus megaterium* HyangYak-01 strain exhibited excellent effects of improving the soil condition and promoting the growth of crops or biosynthesis of biologically active substances, and that the *Centella asiatica* cultivated after treatment with the strain, or the extract thereof exhibited excellent effects of improving a skin condition (e.g., skin barrier strengthening effect) or anti-inflammatory effects.

The description above is merely an illustrative explanation of the technical concept of the present disclosure, and those skilled in the art to which the present disclosure belongs will be able to allow various modifications and changes without departing from the essential characteristics of the present disclosure.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. COSMAX CO., LTD.
46, Jeyakgongdan 2-gil,
Hyangnam-eup, Hwaseong-si,
Gyeonggi-do 18622,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Bacillus megaterium HyangYak-01 | Accession number given by the
INTERNATIONAL DEPOSITARY AUTHORITY:
KCCM13074P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on November. 15. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: November. 15. 2021

¹ Where Rule 6.4(d) applies, such date is the date on which the status of international depositary authority was
acquired.

Form BP/4 (sole page)
한국미생물보존센터
03641 서울특별시 서대문구 홍제내2가길 45 유림빌딩 Tel 02-391-0950, 396-0950 Fax 02-392-2859
KOREAN CULTURE CENTER OF MICROORGANISMS
Yurim Bldg., 45, Hongjenae 2ga-gil, Seodaemun-gu, Seoul, 03641 Korea. Tel 82-2-391-0950, 396-0950 Fax: 82-2-392-2859

## Claims

1. A *Bacillus megaterium* HyangYak-01 strain deposited under Accession No. KCCM13074P.

2. The strain of claim 1, wherein the strain comprises 16S rRNA comprising a base sequence having at least 98 % sequence identity to SEQ ID NO: 1.

3. The strain of claim 1, wherein the strain exhibits effects of improving soil condition, promoting plant growth, or promoting biosynthesis of biologically active substances in plants.

4. The strain of claim 3, wherein the improving of soil condition comprises increasing diversity of microbial species in soil microbiome or improving physiochemical properties of soil.

5. The strain of claim 1, wherein the strain has any one or more of the following activities:
(1) production activity of a root growth-promoting hormone (indole-3-acetic acid: IAA);
(2) nitrogen fixation activity;
(3) phosphate solubilization activity;
(4) urea hydrolysis enzyme (urease) activity; and
(5) denitrification activity.

6. A microbial agent comprising the strain of claim 1, a lysis solution or culture thereof, or an extract of the strain, the lysis solution, or the culture

7. The microbial agent of claim 1, wherein the microbial agent is for improving soil condition, promoting plant growth, or promoting biosynthesis of biologically active substances in plants.

8. A microbial fertilizer comprising the microbial agent of claim 6.

9. The microbial fertilizer of claim 8, wherein the microbial fertilizer is for improving soil condition, promoting plant growth, or promoting biosynthesis of biologically active substances in plants.

10. A method of cultivating a plant, the method comprising treating any one or more of soil, a plant, and a plant seed, with the strain, microbial agent or microbial fertilizer of any one of claims 1 to 9.

11. The method of claim 10, wherein the plant is *Centella asiatica, Angelica giga, Platycodon grandiflorum, Codonopsis lanceolata, Potentilla chinensis, Isodon japonicus,* or *Glycyrrhiza uralensis.*

12. A plant cultivated by the method of claim 10.

13. A cosmetic composition for improving a skin condition, the cosmetic composition comprising *Centella asiatica* cultivated by the method of claim 11 or an extract thereof.

14. The cosmetic composition of claim 13, wherein the *Centella asiatica* has an increased amount of at least one of chlorophyll, madecassic acid, and asiatic acid.

15. The cosmetic composition of claim 13, wherein the *Centella asiatica* or the extract thereof has one or more of the following characteristics:
(1) inducing increased expression of filaggrin (FLG), claudin-1 (CLD1), or a combination thereof; and
(2) inducing inhibition of expression of pro-inflammatory cytokines.

16. The cosmetic composition of claim 13, wherein the improving of a skin condition comprises skin barrier strengthening or anti-inflammation.

17. A topical skin composition comprising the *Centella asiatica* cultivated by the method of claim 11 or an extract thereof.

18. A food composition comprising the *Centella asiatica* cultivated by the method of claim 11 or an extract thereof.

19. A pharmaceutical composition for preventing or treating a skin inflammatory disease, the pharmaceutical composition comprising, as an active ingredient, the *Centella asiatica* cultivated by the method of claim 11 or an extract thereof.

20. The pharmaceutical composition of claim 19, wherein the skin inflammatory disease is any one selected from the group consisting of dermatitis, atopic dermatitis, pruritus, eczematous skin disease, dry eczema, erythema, urticaria, psoriasis, drug rash, and acne.

21. A method of improving a skin condition or preventing or treating a skin inflammatory disease of a subject, the method comprising administering an effective amount of the *Centella asiatica* cultivated by the method of claim 11 or an extract thereof into a subject.

22. Use for preparation of a *Bacillus megaterium* HyangYak-01 strain deposited under Accession No. KCCM13074P, a lysis solution or culture thereof, or an extract of the strain, the lysis solution, or the culture.
